# EUROPEAN PATENT APPLICATION

(11) **EP 1 491 188 A1**
(43) Date of publication of application: **29.12.2004**
(21) Application number: 03014278.0
(22) Date of filing: 25.06.2003
(51) Int. Cl.: A61K 31/19, A61K 31/20, A61K 31/44, A61K 31/4436, A61K 31/505

(54) **Topical use of valproic acid for the prevention or treatment of skin disorders**

(71) Applicant: G2M Cancer Drugs AG, 60596 Frankfurt am Main (DE)
(72) Inventor: Pelicci, Pier Giuseppe, Prof. Dr., 20090 Opera (Milano) (IT); Minucci, Saverio, Prof. Dr., 20090 Opera (Milano) (IT); Costanzo, Antonio, Dr., 00199 Roma (IT); Chimenti, Sergio, Prof. Dr., 00199 Roma (IT); Nisticò, Steven Paul, Dr., 00195 Roma (IT); Paolino, Donatella, Dr., 95030 Pedara (IT)
(74) Representative: Keller, Günter, Dr.

(57) **Abstract**

The present invention relates to a topically applicable formulation containing Valproic Acid or a derivative thereof which can be used alone or in combination with topically applicable formulations of retinoids or of nuclear receptor ligands, or of chemotherapeutic agents (e.g. 5-Fluorouracil). The formulation is useful for the topical treatment of cancerous skin disorders, such as Basal Cell Carcinoma, Squamous Cell Carcinoma, Keratoakantoma, Bowen Disease, cutaneous T-Cell Lymphoma and also for the topical treatment of pre-malignant lesions, and of inflammations of the skin and/or mucosa. The invention also relates to the use of this topically applicable formulation for the protection from UV light and for the treatment of sun burn. The invention includes the use of VPA for the manufacture of a clinically used medicament for the topical treatment of the human diseases listed above.

## Description

The present invention relates to a topically applicable formulation containing Valproic Acid or derivatives thereof which can be used alone or in combination with topically applicable formulations of retinoids or of nuclear receptor ligands, or of chemotherapeutic agents (e.g. 5-Fluorouracil) for the topical treatment of human cancers, such as Basal Cell Carcinoma, Squamous Cell Carcinoma, Keratoakantoma, Bowen Disease, cutaneous T-Cell Lymphoma and also for the topical treatment of pre-malignant lesions (such as Actinic Keratose), and of inflammations of the skin and/or mucosa (such as Psoriasis, Ichtiosis, Acne). The invention also relates to the use of this topically applicable formulation for the protection from UV light and for the treatment of sun burn. Additionally, this formulation can be used in diseases which are associated with hypoacetylation of histones or in which induction of hyperacetylation has a beneficial effect, e.g., by induction of differentiation and/or apoptosis in transformed skin and mucosa cells. Furthermore, the formulation is useful for the treatment of other proliferative diseases of the skin and/or mucosa, and for therapy or prophylaxis of conditions associated with abnormal gene expression in skin and/or mucosa tissue. The invention includes the manufacture of a clinically used medicament for the topical treatment of human diseases listed above.

### BACKGROUND OF THE INVENTION

### Chromatin Regulation and Cancer

Local remodeling of chromatin is a key step in the transcriptional activation of genes. Dynamic changes in the nucleosomal packaging of DNA must occur to allow transcriptional proteins to make contact with the DNA template. One of the most important mechanisms influencing chromatin remodeling and gene transcription are the posttranslational modification of histones and other cellular proteins by acetylation and subsequent changes in chromatin structure (Davie, 1998, Curr Opin Genet Dev 8, 173-8; Kouzarides, 1999, Curr Opin Genet Dev 9, 40-8; Strahl and Allis, 2000, Nature 403, 41-4). In the case of histone hyperacetylation, changes in electrostatic attraction for DNA and steric hindrance introduced by the hydrophobic acetyl group leads to destabilisation of the interaction of histones with DNA. As a result, acetylation of histones disrupts nucleosomes and allows the DNA to become accessible to the transcriptional machinery. Removal of the acetyl groups allows the histones to bind more tightly to DNA and to adjacent nucleosomes, and thus, to maintain a transcriptionally repressed chromatin structure. Acetylation is mediated by a series of enzymes with histone acetyltransferase (HAT) activity. Conversely, acetyl groups are removed by specific histone deacetylase (HDAC) enzymes. Disruption of these mechanisms gives rise to transcriptional misregulation and may contribute to tumorigenic transformation and tumor progression.

Additionally, other molecules such as transcription factors alter their activity and stability depending on their acetylation status. E.g. PML-RAR, the fusion protein associated with acute promyelocytic leukemia (APL) inhibits p53 through mediating deacetylation and degradation of p53, thus allowing APL blasts to evade p53 dependent cancer surveillance pathways. Expression of PML-RAR in hematopoietic precursor cells results in repression of p53 mediated transcriptional activation, and protection from p53-dependent apoptosis triggered by genotoxic stresses (X-rays, oxidative stress). However, the function of p53 is reinstalled in the presence of HDAC inhibitors implicating active recruitment of HDAC to p53 by PML-RAR as the mechanism underlying p53 inhibition (Insinga et al. 2003, manuscript submitted). Therefore, acetylation of proteins distinct from histones, such as acetylation of p53, plays a crucial role in the anti-tumor activity of HDAC inhibitors.

### Nuclear Receptors and Histone Deacetylases

Nuclear hormone receptors are ligand-dependent transcription factors that control development and homeostasis through both positive and negative control of gene expression. Defects in these regulatory processes underlie the causes of many diseases and play an important role in the development of cancer. Many nuclear receptors, including T3R, RAR and PPAR, can interact with corepressors, such as N-CoR and SMRT, in the absence of ligand and thereby inhibit transcription. Furthermore, N-CoR has also been reported to interact with antagonist-occupied progesterone and estrogen receptors. Most interestingly, N-CoR and SMRT have been shown to exist in large protein complexes, which also contain mSin3 proteins and histone deacetylases (Pazin and Kadonaga, 1997; Cell 89, 325-8). Thus, the ligand-induced switch of nuclear receptors from repression to activation reflects the exchange of corepressor and coactivator complexes with antagonistic enzymatic activities.

### Gene Regulation by Nuclear Receptors

Such corepressor complexes which contain HDAC activity, not only mediate repression by nuclear receptors, but also interact with additional transcription factors including Mad-1, BCL-6 and ETO. Many of these proteins play key roles in disorders of cell proliferation and differentiation (Pazin and Kadonaga, 1997, Cell 89, 325-8; Huynh and Bardwell, 1998, Oncogene 17, 2473-84; Wang, J. et al., 1998, Proc Natl Acad Sci U S A 95, 10860-5). T3R for example was originally identified on the basis of its homology with the viral oncogene v-erbA, which in contrast to the wild type receptor does not bind ligand and functions as a constitutive repressor of transcription. Furthermore, mutations in RARs have been associated with a number of human cancers, particularly acute promyelocytic leukemia (APL) and hepatocellular carcinoma. In APL patients RAR fusion proteins resulting from chromosomal translocations involve either the promyelocytic leukemia protein (PML) or the promyelocytic zinc finger protein (PLZF). Although both fusion proteins can interact with components of the corepressor complex, the addition of retinoic acid dismisses the corepressor complex from PML-RAR, whereas PLZF-RAR interacts constitutively. These findings provide an explanation why PML-RAR APL patients achieve complete remission following retinoic acid treatment whereas PLZF-RAR APL patients respond very poorly (Grignani et al., 1998, Nature 391, 815-8, Guidez et al., 1998, Blood 91, 2634-42; He et al., 1998, Nat Genet 18, 126-35; Lin et al., 1998, Nature 391, 811-4).

Recently, a PML-RAR patient who had experienced multiple relapses after treatment with retinoic acid has recently been treated with the HDAC inhibitor phenylbutyrate, resulting in complete remission of the leukemia (Warrell et al., 1998, J. Natl. Cancer Inst. 90, 1621-1625).

### Anti-Cancer Therapy with HDAC Inhibitors

Additional clinical investigations have recently been initiated to exploit the systemic clinical treatment of cancer patients with the principle of HDAC inhibition. By now, a clinical phase II trial with the closely related butyric acid derivative Pivanex (Titan Pharmaceuticals) as a monotherapy has been completed demonstrating activity in stage III/IV non-small cell lung cancer (Keer et al., 2002, ASCO, Abstract No. 1253). More HDAC inhibitors have been identified, with NVP-LAQ824 (Novartis) and SAHA (Aton Pharma Inc.) being members of the structural class of hydroxamic acids tested in phase II clinical trials (Marks et al., 2001, Nature Reviews Cancer 1, 194-202). Another class comprises cyclic tetrapeptides, such as depsipeptide (FR901228 - Fujisawa) used successfully in a phase II trial for the treatment of T-cell lymphomas (Piekarz et al., 2001, Blood 98, 2865-8). Furthermore, MS-27-275 (Mitsui Pharmaceuticals), a compound related to the class of benzamides, is now being tested in a phase I trial treating patients with hematological malignancies.

### The Protein Family of Histone Deacetylases

Mammalian histone deacetylases can be divided into three subclasses (Gray and Ekström, 2001). HDACs 1, 2, 3, and 8 which are homologues of the yeast RPD3 protein constitute class I. HDACs 4, 5, 6, 7, 9, and 10 are related to the yeast Hda 1 protein and form class II. Recently, several mammalian homologues of the yeast Sir2 protein have been identified forming a third class of deacetylases which are NAD dependent. All of these HDACs appear to exist in the cell as subunits of a plethora of multiprotein complexes. In particular, class I and II HDACs have been shown to interact with transcriptional corepressors mSin3, N-CoR and SMRT which serve as bridging factors required for the recruitment of HDACs to transcription factors.

### Valproic Acid

Valproic acid (VPA; 2-propyl-pentanoic acid) has multiple biological activities which depend on different molecular mechanisms of action:
- VPA is an antiepileptic drug.
- VPA is teratogenic. When used as an antiepileptic drug during pregnancy, VPA can induce birth defects (neural tube closure defects and other malformations) in a few percent of born children. In mice, VPA is teratogenic in the majority of mouse embryos when properly dosed.
- VPA activates a nuclear hormone receptor (PPARδ). Several additional transcription factors are also derepressed but some factors are not significantly derepressed (glucocorticoid receptor, PPARα).
- VPA occasionally causes hepatotoxicity, which may depend on poorly metabolized esters with coenzyme A.
- VPA is an inhibitor of HDACs.

The use of VPA derivatives allowed to determine that the different activities are mediated by different molecular mechanisms of action. Teratogenicity and antiepileptic activity follow different modes of action because compounds could be isolated which are either preferentially teratogenic or preferentially antiepileptic (Nau et al., 1991, Pharmacol. Toxicol. 69, 310-321). Activation of PPARδ was found to be strictly correlated with teratogenicity (Lampen et al., 1999, Toxicol, Appl. Pharmacol. 160, 238-249) suggesting that, both, PPARδ activation and teratogenicity require the same molecular activity of VPA. Also, differentiation of F9 cells strictly correlated with PPARδ activation and teratogenicity as suggested by Lampen et al., 1999, and documented by the analysis of differentiation markers (Werling et al., 2001, Mol. Pharmacol. 59, 1269-1276). It was shown, that PPARδ activation is caused by the HDAC inhibitory activity of VPA and its derivatives (PCT/EP01/07704; WO 03/024442 A2). Furthermore, it was shown that the established HDAC inhibitor TSA activates PPARδ and induces the same type of F9 cell differentiation as VPA. From these results it can be concluded that not only activation of PPARδ but also induction of F9 cell differentiation and teratogenicity of VPA or VPA derivatives are caused by HDAC inhibition.

Antiepileptic and sedating activities follow different structure activity relationships and thus obviously depend on a primary VPA activity distinct from HDAC inhibition. The mechanism of hepatotoxicity is poorly understood, and it is unknown whether it is associated with formation of the VPA-CoA ester. HDAC inhibition, however, appears not to require CoA ester formation.

### Retinoic Acid

Retinoic Acid (RA) belongs to a class of compounds known as retinoids, including but not limited to 9-cis RA, trans RA, all-trans RA, Tazaratone (Guenther, 2003, Am. J. Clin. Dermatol. 4, 197-202; Peris K et al., 1999, N Engl J Med 341, 1767-1768), that play key roles in regulating gene transcription and, therefore, govern multiple functions in the body, such as cell division and differentiation, immune response and embryonic development. They also control the development and spread of cancer cells, and some, including RA, can inhibit tumor growth by preventing cancer cell proliferation (Altucci and Gronemeyer, 2001, Nature Reviews Cancer 1, 181-193).

The effects of retinoids are mainly mediated by two classes of nuclear receptors, the RA receptors (RARs) and retinoid X receptors (RXRs) (Zhang & Pfahl 1993, Kastner *et al.* 1995, Mangelsdorf & Evans 1995). RARs and RXRs are encoded by three distinct genes (α, β and γ). In addition, many retinoid receptor isoforms are generated through differential promoter usage, giving rise to a large number of distinct retinoid receptor proteins. To date, there are dozens of receptors which are known to mediate the effect of retinoids. 9-*cis* RA is a high-affinity ligand for both RARs and RXRs, whereas *trans*-RA is a ligand for only RARs. Retinoid receptors belong to a large steroid/thyroid receptor superfamily that mediate the biological effects of many hormones, vitamins and drugs. RARs and RXRs act as transcriptional factors to positively or negatively regulate expression of target genes by binding to their response elements (RAREs) located in promoter regions of the target genes of an active chromatin structure and results in the transcription of target genes. In addition to their direct effects on transcription, liganded RAR can modulate the activity of other transcriptional factors, such as AP-1 (Pfahl 1993). Activated retinoid receptors can inhibit the activity of AP-1, thereby regulating the expression of AP-1 target genes. The inhibition of AP-1 activity is linked to the anti-proliferative effects of retinoids, and appears to be separable from their direct activation of transcription of retinoid-target genes.

For some time doctors have been using RA derivatives, to treat several cancers, particularly prostate cancer and leukemia, and they are now experimenting with the drug to treat breast cancer. A typical treatment with RA seeks to activate RAR in order to switch on favorable genes. The great drawback to RA, however, is that it requires high levels of the medication in order to turn genes 'on' and 'off,' often triggering devastating and potentially fatal side effects (Altucci and Gronemeyer, 2001, Nature Reviews Cancer 1, 181-193).

RA or other retinoids such as Tazarotene (Guenther, 2003, Am. J. Clin. Dermatol. 4, 197-202) may also be used topically for the treatment of mild to moderate acne and sun damage (photoaged) skin. Retinoids have even been used to treat certain skin cancers, such as Basal Cell Carcinoma (Peris K et al., 1999, N Engl J Med 341, 1767-1768). After topical administration, RA appears to increase the rate of cell division and turnover. The number of cell layers in the outer portion of the skin is decreased. When pimples are present, they are more rapidly resolved. Topical RA is effective in reducing fine wrinkling, mottled hyperpigmentation, and roughness associated with overexposure to the sun. The results of treatment are not immediate and may be visible only after weeks to months. Discontinuation of treatment usually results in a loss of RA effects.

### Basal Cell Carcinoma

Basal cell carcinoma (BCC) is not only the most common form of skin cancer, it is also the most common of all cancers affecting, e.g., 800,000 US Americans each year. These cancers arise in the basal cells, which are at the bottom of the epidermis (outer skin layer). Although the number of new cases has increased sharply each year in the last few decades, the average age of onset of the disease has steadily decreased. Until recently, those most often affected were older people, particularly men who had worked outdoors. However, today more women are getting BCC's than in the past; nonetheless, men still outnumber them greatly.

Chronic exposure to sunlight is the cause of almost all basal cell carcinomas, which occur most frequently on exposed parts of the body - the face, ears, neck, scalp, shoulders, and back. People who have fair skin, light hair, and blue, green, or gray eyes are at highest risk. Those whose occupations require long hours outdoors or who spend extensive leisure time in the sun are in particular jeopardy. Predisposing factors for BCC include ultraviolet radiation, ionizing radiation, arsenic, inherited conditions, such as xeroderma pigmentosum and basal cell nevus syndrome, vaccinations, burns, and scars.

The typical lesion is a smooth-surfaced nodule with raised, pearly or translucent borders. Telangiectasias in the lesion are a common feature. Basal cell carcinomas vary in size from a few millimeters to several centimeters. Size can double in 6-12 months or growth may be slow. Eighty five percent occur on the head and neck with the nose being the most common site. Patients may report bleeding with minor trauma such as washing with a face cloth. This neoplasm is locally invasive and can be very destructive causing the loss of an eye, ear, or a nose, and may be lethal if it invades the brain. BCC rarely metastasizes but when it does it can be by way of the lymphatics to regional lymph nodes, or by hematogenous spread most commonly to the lungs. Advanced skin cancer may be arbitrarily defined as tumors > 2cm, with invasion of bone muscle, or nerves, lymph node metastasis, or lesions that require removal of a cosmetic or functional unit.

The treatment for a BCC depends on its type, size and location, the number to be treated, and the preference or expertise of the doctor. Possibilities include:
- Excision - the lesion is cut out and the skin stitched up.
- Freezing (cryotherapy) - use of liquid nitrogen for small superficial lesions. This is not a recommended treatment for BCC.
- Curettage and cautery - scraping of the tumor and cauterising the base. This is also not a recommended treatment for BBC.
- Radiotherapy (X-ray treatment) - can be used in advanced lesions or where surgery is not suitable.
- Topical chemotherapy - topical application of a cream containing 5-Fluorouracil (5-FU) can be used especially for very large regions not treatable by surgery.

However, for those patients unwilling or unsuitable for surgery (e.g., the frail and elderly, those with a pacemaker or with a bleeding diathesis) or with large lesions, multiple lesions and those on anatomically difficult sites or poor vascularized skin, there remains a need for additional effective treatments.

Today, tumor therapies are known which consist of the combinatorial treatment of patients with more than one anti-tumor therapeutic reagent. Examples are the combined use of irradiation treatment together with chemotherapeutic and/or cytotoxic reagents and more recently the combination of irradiation treatment with immunological therapies, such as the use of tumor cell specific therapeutic antibodies. However, the possibility to combine individual treatments with each other in order to identify such combinations which are more effective than the individual approaches alone, requires extensive pre-clinical and clinical testing. It is not possible to predict which combinations will show an additive or even synergistic effect. Besides the aim to increase the therapeutic efficacy, another purpose is the potential decrease of the doses of the individual components in the resulting combinations in order to decrease unwanted or harmful side effects caused by higher doses of the individual components.

The present invention aims at providing an improved composition for the prevention or treatment of skin disorders.

VPA has been developed as a drug used for the treatment of epilepsia. Accordingly, VPA is used systemically, orally or intravenously, to allow the drug to pass the blood brain barrier to reach the epileptic target regions in the brain tissue in order to fulfill its anti-epileptic mission. Therefore, VPA is regarded as a drug that must be applied systemically in order to achieve its therapeutic effects.
Now, it was surprisingly identified that VPA permeates human skin effectively, which is crucially required for any therapeutic compound to be used topically on skin. It was hypothesized by the inventors that VPA, by fulfilling this essential criteria for topical application, could thus be used for the topical treatment of cancer lesions of the skin.

To this end, it was now found that VPA has in fact unexpected beneficial effects when used for the topical treatment of human skin diseases, e.g., human skin cancer. Here, the precise mode of action which is employed by VPA is not fully understood, but its potential to sensitize tumor cells for the activity of apoptosis, growth arrest and differentiation inducing compounds may be the basis of such an anti-tumor activity. This surprising potential of VPA is expected to be based on its activity as an inhibitor of specific sets of enzymes having HDAC activity.

The invention therefore relates to a topical pharmaceutical composition for the prevention or treatment of skin disorders, comprising:
(i) at least about 0.1% of an active agent selected from the group consisting of VPA and pharmaceutically acceptable salts thereof, derivatives of VPA and pharmaceutically acceptable salts thereof; and
(ii) a dermatologically acceptable carrier.

The term "topical application", as used herein, means to apply or spread the compositions of the present invention onto the surface of the skin.

The term "dermatologically acceptable," as used herein, means that the compositions or components thereof so described are suitable for use in contact with human skin without undue toxicity, incompatibility, instability, allergic response, and the like.

Unless indicated otherwise, percentage values given herein are % by weight (w/w).

The compositions of the present invention preferably comprise from about 0.1% to about 25%, more preferably from about 0.1 % to about 6%, even more preferably from about 0.3% to about 5%, still more preferably from about 0.5% to about 4%, still more preferably from about 1% to about 4%, most preferably from about 2% to about 4%, of the active agent.

The compositions of the present invention usually comprise from about 1% to about 99.9% of a dermatologically acceptable carrier within which the compositions of the present invention is incorporated to enable the active agent, as well as other optional actives, to be delivered to the skin at an appropriate concentration.

In a preferred embodiment the composition of the invention is a semisolid at 25°C and under atmospheric pressure. In accordance with this embodiment, the product form of the composition may be a cream, an ointment, a gel or a paste. The product form of the composition may be a liquid dispersion, e.g. a lotion.

Unexpectedly, it has been found that topical application of VPA potentiates the therapeutic effect of retinoids and chemotherapeutic drugs such as 5-Fluorouracil.

In another aspect of the invention the composition therefore further comprises retinoic acid or a derivative thereof. The concentration of retinoic acid or the derivative in the composition is preferably from about 0.01% to about 1%, more preferably from about 0.05% to about 0.5% of the composition. The retinoid is preferably selected from the group consisting of 9-cis retinoic acid, trans-retinoic acid, all-trans retinoic acid and Tazarotene.

In yet another aspect of the invention the composition further comprises a chemotherapeutic drug such as 5-Fluorouracil. The concentration of the chemotherapeutic drug preferably is from about 0.1% to about 10%, more preferably from about 1% to about 10% of the composition.

In a preferred embodiment, the carrier is not a solution. In another preferred embodiment, the carrier is a cream, a paste, an ointment, a lotion or a gel.

Another aspect of the invention is the use of VPA, a pharmaceutically acceptable salt thereof, a derivative of VPA or a pharmaceutically acceptable salt thereof for the manufacture of a medicament for the prevention or treatment of a skin disorder wherein a medicament comprising at least about 0.1 % VPA or derivative thereof is topically applied to the skin of an individual in need thereof.

The skin disorder preferably is a disease of the human skin in which induction of hyperacetylation of proteins has a beneficial therapeutic effect for patients. The skin disorder may be a skin tumor, e.g., Basal Cell Carcinoma, Squamous cell carcinoma, Keratoakantoma, Bowen disease and cutaneous T-Cell Lymphoma.

The skin disorder may be a pre-neoplastic skin disease such as Actinic keratosis. In other embodiments, VPA or its derivative may be used for the treatment of inflammations of the skin and/or mucosa. Non-limiting examples of inflammations of the skin and/or mucosa are Psoriasis, Ichtiosis and Acne. The skin disorder may also be sun damaged skin (photoaged skin, sun burn).

Administration of VPA or the derivative thereof according to the present invention may be combined with an established anti-cancer therapy. VPA or the derivative thereof and the established cancer therapy may be applied simultaneously or successively (at different time points).

Further optional actives may be used in the treatment according to the invention. VPA or the derivative thereof and the further active may be administered simultaneously or successively (at different time points). Further actives include inhibitors of histone deacetylases which are different to VPA, including but not limited to compounds such as NVP-LAQ824 (Novartis), Trichostatin A, Suberoyl anilide hydroxamic acid (Aton), CBHA (ATON), Pyroxamide (Aton), Scriptaid (Johns Hopkins), Cl-994 (Pfizer), CG-1521 (CircaGen), Chlamydocin (Janssen), Biaryl hydroxamate, e.g., A-161906 (Abbott), Bicyclic aryl-N-hydroxycarboxamides (Kansai University), PXD-101 (Prolifix), Sulfonamide hydroxamic acid (MethylGene), TPX-HA analogue (CHAP) (Japan Energy), Oxamflatin, Trapoxin, Depudecin, Apidicin (Kyongji), benzamides such as MS-27-275 (Mitsui), pyroxamides and derivatives therof, short chain fatty acids such as butyric acid, and derivatives thereof, e.g., Pivanex (Pivaloyloxymethyl butyrate), cyclic tetrapeptides such as trapoxin A, Depsipeptide (FK-228; Fujisawa/NCl) and related peptidic compounds, Tacedinaline (Pfizer), MG2856 (MethylGene), and HDAC class III inhibitors or SIRT inhibitors (see Kelly, O'Connor and Marks, 2002; Expert Opin. Inverstig. Drugs 11 (12), 1695-1713).

Administration of VPA or the derivative thereof may be combined with administration/application of chemotherapeutic or cytotoxic drugs (e.g. 5-FU), differentiation inducing drugs (e.g. vitamin D, vitamin D derivatives, retinoids, receptor binding agents such as imiquimode), radiation therapy (e.g. x-rays or gamma rays), immunological approaches (antibody therapy, vaccination), combined immunotherapeutic/cytotoxic approaches (e.g. antibodies conjugated with cytotoxic components), and the like.

In a further embodiment, VPA or its derivative potentiates orally applied retinoid activity in neoplastic and non-neoplastic skin diseases including skin tumors; Basal Cell Carcinoma; Squamous cell carcinoma; Keratoakantoma; Bowen disease; cutaneous T-Cell Lymphoma; Actinic keratosis; Psoriasis; Ichtiosis; Acne; other inflammatory skin diseases by topical application of VPA alone.

The invention provides a topically applicable formulation of a medicament containing Valproic Acid or derivatives thereof used alone or in combination with retinoids, with nuclear receptor ligands or with chemotherapeutic agents, such as 5-FU, for the topical treatment of cancer, pre-malignant skin lesions, and inflammations of the skin/mucosa.

Therefore, one aspect of the present invention is the use of a topically applicable formulation containing VPA or derivatives thereof used alone or in combination with retinoids or with 5-Fluorouracil (5-FU) for a topical treatment of a variety of human cancers, pre-malignant skin lesions or inflammations of the skin/mucosa, and for the treatment of sun burn. The antitumoral activity of combinatorial topical treatments compared to the use of each component alone can thus be increased and - if desired - the doses of the individual components of such combinatorial treatments may be lowered in order to decrease unwanted side effects related to treatment with the individual drugs alone. The invention also concerns the use of VPA or a derivative thereof for the manufacture of a medicament for a topical treatment of diseases as listed above.

The derivatives of VPA are defined as carbon branched carboxylic acids or carboxylic acid derivatives as described by formula I 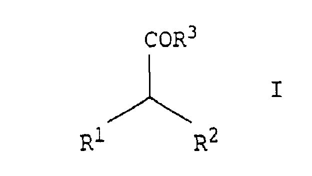 wherein R¹ and R² independently are a linear or branched, saturated or unsaturated aliphatic C₃₋₂₅ hydrocarbon chain which optionally comprises one or several heteroatoms and which may be substituted, R³ is hydroxyl, halogen, alkoxy or an optionally alkylated amino group.

Different R¹ and R² residues give rise to chiral compounds. Usually one of the stereoisomers has a stronger teratogenic effect than the other one (Nau et al., 1991, Pharmacol. Toxicol. 69, 310-321) and the more teratogenic isomer more efficiently activates PPARδ (Lampen et al, 1999). Therefore, this isomer can be expected to inhibit HDACs more strongly (PCT/EP01/07704). The present invention encompasses the racemic mixtures of the respective compounds and in particular the more active isomers.

The hydrocarbon chains R¹ and R² may comprise one or several heteroatoms (e.g. O, N, S) replacing carbon atoms in the hydrocarbon chain. This is due to the fact that structures very similar to that of carbon groups may be adopted by heteroatom groups when the heteroatoms have the same type of hybridization as a corresponding carbon group.

R¹ and R² may be substituted. Possible substituents include hydroxyl, amino, carboxylic and alkoxy groups as well as aryl and heterocyclic groups.

Preferably, R¹ and R² independently comprise 3 to 10, more preferably 4 to 10 or 5 to 10 carbon atoms. It is also preferred that R¹ and R² independently are saturated or comprise one double bond or one triple bond. In particular, one of the side chains (R¹) may preferably contain sp¹ hybridized carbon atoms in position 2 and 3 or heteroatoms which generate a similar structure. This side chain should comprise 3 carbon or heteroatoms but longer chains may also generate HDAC-inhibiting molecules. Also, inclusion of aromatic rings or heteroatoms in R² is considered to generate compounds with HDAC inhibitory activity because the catalytic site of the HDAC protein apparently accommodates a wide variety of binding molecules. With the observation that teratogenic VPA derivatives are HDAC inhibitors, also compounds which have previously been disregarded as suitable antiepileptic agents are considered as HDAC inhibitors (PCT/EP01107704). In particular, but not exclusively, compounds having a propinyl residue as R¹ and residues of 7 or more carbons as R², are considered (Lampen et al, 1999).

Preferably, the group "COR³" is a carboxylic group. Also derivatization of the carboxylic group has to be considered for generating compounds with potential HDAC inhibitory activity. Such derivatives may be halides (e.g. chlorides), esters or amides. When R³ is alkoxy, the alkoxy group comprises 1 to 25, preferably 1-10 carbon atoms. When R³ is a mono- or di-alkylated amino group, the alkyl substituents comprise 1 to 25, preferably 1-10 carbon atoms.

According to the present invention also pharmaceutically acceptable salts of a compound of formula I can be used for the formulation. According to the present invention also substances can be used which are metabolized to a compound as defined in formula I in the human organism or which lead to the release of a compound as defined in formula I for example by ester hydrolysis.

In a particular embodiment, the invention concerns the use of an α-carbon branched carboxylic acid as described in formula I or of a pharmaceutically acceptable salt thereof as an inhibitor of an enzyme having histone deacetylase activity and its use in the topical therapy of cancer, wherein R¹ is a linear or branched, saturated or unsaturated, aliphatic C₅. ₂₅ hydrocarbon chain, R² independently is a linear or branched, saturated or unsaturated, aliphatic C₂₋₂₅ hydrocarbon chain, but not -CH₂-CH=CH₂, -CH₂-C≡CH or -CH₂-CH₂-CH₃, R¹ and R² are optionally substituted with hydroxyl, amino, carboxylic, alkoxy, aryl and/or heterocyclic groups, and R³ is hydroxyl.

In yet another embodiment the invention concerns the use of an α-carbon branched carboxylic acid as described in formula I or of a pharmaceutically acceptable salt thereof for the topical therapy of cancer, wherein R¹ is a linear or branched, saturated or unsaturated, aliphatic C₃₋₂₅ hydrocarbon chain, and R² independently is a linear or branched, saturated or unsaturated, aliphatic C₃₋₂₅ hydrocarbon chain, R¹ or R² comprise one or several heteroatoms (e.g. O, N, S) replacing carbon atoms in the hydrocarbon chain, R¹ and R² are optionally substituted with hydroxyl, amino, carboxylic, alkoxy, aryl and/or heterocyclic groups, and R³ is hydroxyl.

In yet another embodiment of the invention R' and R² do not comprise an ester group (-CO-O-). R¹ and R² may be hydrocarbon chains comprising no heteroatoms O, N or S.

The compounds which are most preferably used according to the present invention are VPA and/or 4-yn VPA.

In general the present invention provides novel possibilities to treat various human diseases. Applicants found that the HDAC inhibitory and cellular differentiation-inducing activity of compounds of formula I can be used successfully alone or in combination with well established and clinically used therapeutic drugs for the topical treatment of tumor cells, including such cells derived from skin tumors and from cutaneous T cell Lymphomas, as well as for pre-malignant skin lesions, and for inflammatory diseases of the skin/mucosa. A combinatorial treatment is considered to generate superior therapeutic success in patients than the corresponding therapeutic drugs used on their own. It is one object of the present invention to provide combinatorial therapeutic approaches using the presented compounds for the topical treatment of cutaneous appearing cancers. Such combinatorial treatments could result in a decrease of the therapeutic doses of, e.g., chemotherapeutic reagents required and could, thus, limit the currently observed, partly very serious side effects of frequently used therapies.

Aspects of the present invention include the combination of compounds of formula I with, but not restricted to, therapeutic principles currently in clinical use or in clinical development, such as
- chemotherapeutic or cytotoxic drugs (e.g. 5-FU)
- differentiation inducing drugs (e.g. vitamin D, vitamin D derivatives, retinoids, receptor binding agents such as imiquimode)
- Radiation therapy (e.g. x-rays or gamma rays)
- immunological approaches (antibody therapy, vaccination)
- combined immunotherapeutic/cytotoxic approaches (e.g. antibodies conjugated with cytotoxic components)
- anti-angiogenesis approaches.

### Formulation

The compositions of the present invention comprise from about 1% to about 99.9% of a dermatologically acceptable carrier within which the compositions of the present invention is incorporated to enable the active agent, as well as other optional actives, to be delivered to the skin at an appropriate concentration.

The carrier may contain one or more dermatologically acceptable solid, semi-solid or liquid fillers, diluents, solvents, extenders and the like. The carrier may be solid, semi-solid or liquid. Preferred carriers are substantially semi-solid. The carrier can itself be inert or it can possess dermatological benefits of its own. Concentrations of the carrier can vary with the carrier selected and the intended concentrations of the active agent and optional components.

Suitable carriers include conventional or otherwise known carriers that are dermatologically acceptable. The carrier should also be physically and chemically compatible with the essential components described herein, and should not unduly impair stability, efficacy or other use benefits associated with the compositions of the present invention. Preferred components of the compositions of this invention should be capable of being coming led in a manner such that there is no interaction which would substantially reduce the efficacy of the composition under ordinary use situations.

The type of carrier utilized in the present invention depends on the type of product form desired for the composition. The topical compositions useful in the subject invention may be made into a wide variety of product forms such as are known in the art. These include, but are not limited to, lotions, creams, gels, sticks, sprays, ointments, oils, foams, powders and pastes. These product forms may comprise several types of carriers including, but not limited to, solutions, aerosols, emulsions, gels, solids, and liposomes.

Preferred carriers contain a dermatologically acceptable, hydrophilic diluent. As used herein, "diluent" includes materials in which the particulate material can be dispersed, dissolved, or otherwise incorporated. Nonlimiting examples of hydrophilic diluents are water, organic hydrophilic diluents such as lower monovalent alcohols (e.g., C₁-C₄) and low molecular weight glycols and polyols, including propylene glycol, polyethylene glycol (e.g., Molecular Weight 200-600 g/mole), polypropylene glycol (e.g., Molecular Weight 425-2025 g/mole), glycerol, butylene glycol, 1,2,4-butanetriol, sorbitol esters, 1,2,6-hexanetriol, ethanol, isopropanol, sorbitol esters, butanediol, ether propanol, ethoxylated ethers, propoxylated ethers and combinations thereof. Water is a preferred diluent. The composition preferably comprises from about 60% to about 99.99% of the hydrophilic diluent.

Solutions according to the subject invention typically include a dermatologically acceptable hydrophilic diluent. Solutions useful in the subject invention preferably contain from about 60% to about 99.99% of the hydrophilic diluent.

Aerosols according to the subject invention can be formed by adding a propellant to a solution such as described above. Exemplary propellants include chloro-fluorinated lower molecular weight hydrocarbons. Aerosols are typically applied to the skin as a spray-on product.

Preferred carriers comprise an emulsion comprising a hydrophilic phase comprising a hydrophilic component, e.g., water or other hydrophilic diluent, and a hydrophobic phase comprising a hydrophobic component, e.g., a lipid, oil or oily material. As well known to one skilled in the art, the hydrophilic phase will be dispersed in the hydrophobic phase, or vice versa, to form respectively hydrophilic or hydrophobic dispersed and continuous phases, depending on the composition ingredients. In emulsion technology, the term "dispersed phase" is a term well-known to one skilled in the art which means that the phase exists as small particles or droplets that are suspended in and surrounded by a continuous phase. The dispersed phase is also known as the internal or discontinuous phase. The emulsion may be or comprise (e.g., in a triple or other multi-phase emulsion) an oil-in-water emulsion or a water-in-oil emulsion such as a water-in-silicone emulsion. Oil-in-water emulsions typically comprise from about 1% to about 50% of the dispersed hydrophobic phase and from about 1% to about 98% of the continuous hydrophilic phase; water-in-oil emulsions typically comprise from about 1% to about 98% of the dispersed hydrophilic phase and from about 1% to about 50% of the continuous hydrophobic phase. The emulsion may also comprise a gel network. Preferred emulsions are further described below.

The topical compositions of the subject invention, including but not limited to lotions and creams, may comprise a dermatologically acceptable emollient. Such compositions preferably contain from about 2% to about 50% of the emollient. Emollients tend to lubricate the skin, increase the smoothness and suppleness of the skin, prevent or relieve dryness of the skin, and/or protect the skin. Emollients are typically water-immiscible,.oily or waxy materials. Non-limiting examples of emollients are described herein.

Lotions and creams according to the present invention preferably comprise a solution carrier system and one or more emollients. Lotions typically comprise from about 1% to about 20%, preferably from about 5% to about 10%, of emollient; from about 50% to about 90%, preferably from about 60% to about 80%, water. A cream typically comprises from about 5% to about 50%, preferably from about 10% to about 20%, of emollient; and from about 45% to about 85%, preferably from about 50% to about 75%, water.

Ointments of the present invention may comprise a simple carrier base of animal or vegetable oils or semi-solid hydrocarbons (oleaginous); absorption ointment bases which absorb water to form emulsions; or water soluble carriers, e.g., a water soluble solution carrier. Ointments may further comprise a thickening agent. For example, an ointment may comprise from about 2% to about 10% of an emollient; and from about 0.1% to about 2% of a thickening agent. Non-limiting examples of thickening agents are described herein.

Preferred topical compositions of the present invention comprise an emulsion. Emulsions of the present invention may contain one or more of the following:

### a) Hydrophobic component

Emulsions according to the present invention contain a hydrophobic phase comprising a lipid, oil, oily or other hydrophobic component. The compositions of the present invention preferably comprise from about 1% to about 50%, preferably from about 1% to about 30%, and more preferably from about 1% to about 10% by weight of the composition of a hydrophobic component. The hydrophobic component may be derived from animals, plants, or petroleum and may be natural or synthetic (i.e., man-made). Preferred hydrophobic components are substantially water-insoluble, more preferably essentially water-insoluble.

Nonlimiting examples of suitable hydrophobic components include those selected from the group consisting of:
(1) Mineral oil, which is also known as petrolatum liquid, is a mixture of liquid hydrocarbons obtained from petroleum. See The Merck Index, Tenth Edition, Entry 7048, p. 1033 (1983).
(2) Petrolatum, which is also known as petroleum jelly, is a colloidal system of nonstraight-chain solid hydrocarbons and high-boiling liquid hydrocarbons, in which most of the liquid hydrocarbons are held inside the micelles. See The Merck Index, Tenth Edition, Entry 7047, p. 1033 (1983); Schindler, Drug. Cosmet. Ind., 89, 36-37, 76, 78-80, 82 (1961).
(3) Straight and branched chain hydrocarbons having from about 7 to about 40 carbon atoms. Nonlimiting examples of these hydrocarbon materials include dodecane, isododecane, squalane, cholesterol, hydrogenated polyisobutylene, docosane, hexadecane, isohexadecane. Also useful are the C7-C40 isoparaffins, which are C7-C40 branched hydrocarbons, e.g., C13-C14 isoparaffin.
(4) C1-C30 alcohol esters of C1-C30 carboxylic acids and of C2-C30 dicarboxylic acids, including straight and branched chain materials as well as aromatic derivatives (as used herein in reference to the hydrophobic component, mono- and poly-carboxylic acids include straight chain, branched chain and aryl carboxylic acids). Nonlimiting examples include diisopropyl sebacate, diisopropyl adipate, isopropyl myristate, isopropyl palmitate, methyl palmitate, myristyl propionate, 2-ethylhexyl palmitate, isodecyl neopentanoate, di-2-ethylhexyl maleate, cetyl palmitate, myristyl myristate, stearyl stearate, isopropyl stearate, methyl stearate, cetyl stearate, behenyl behenrate, dioctyl maleate, dioctyl sebacate, diisopropyl adipate, cetyl octanoate, diisopropyl dilinoleate.
(5) mono-, di- and tri-glycerides of C1-C30 carboxylic acids, e.g., caprilic/capric triglyceride, PEG-6 caprylic/capric triglyceride, PEG-8 caprylic/capric triglyceride.
(6) alkylene glycol esters of C1-C30 carboxylic acids, e.g., ethylene glycol mono- and di-esters, and propylene glycol mono- and di-esters of C1-C30 carboxylic acids e.g., ethylene glycol distearate.
(7) propoxylated and ethoxylated derivatives of the foregoing materials.
(8) C1-C30 mono- and poly-esters of sugars and related materials. These esters are derived from a sugar or polyol moiety and one or more carboxylic acid moieties. Depending on the constituent acid and sugar, these esters can be in either liquid or solid form at room temperature. Examples of liquid esters include: glucose tetraoleate, the glucose tetraesters of soybean oil fatty acids (unsaturated), the mannose tetraesters of mixed soybean oil fatty acids, the galactose tetraesters of oleic acid, the arabinose tetraesters of linoleic acid, xylose tetralinoleate, galactose pentaoleate, sorbitol tetraoleate, the sorbitol hexaesters of unsaturated soybean oil fatty acids, xylitol pentaoleate, sucrose tetraoleate, sucrose pentaoletate, sucrose hexaoleate, sucrose hepatoleate, sucrose octaoleate, and mixtures thereof. Examples of solid esters include: sorbitol hexaester in which the carboxylic acid ester moieties are palmitoleate and arachidate in a 1:2 molar ratio; the octaester of raffinose in which the carboxylic acid ester moieties are linoleate and behenate in a 1:3 molar ratio; the heptaester of maltose wherein the esterifying carboxylic acid moieties are sunflower seed oil fatty acids and lignocerate in a 3:4 molar ratio; the octaester of sucrose wherein the esterifying carboxylic acid moieties are oleate and behenate in a 2:6 molar ratio; and the octaester of sucrose wherein the esterifying carboxylic acid moieties are laurate, linoleate and behenate in a 1:3:4 molar ratio. A preferred solid material is sucrose polyester in which the degree of esterification is 7-8, and in which the fatty acid moieties are C18 mono- and/or di-unsaturated and behenic, in a molar ratio of unsaturates:behenic of 1:7 to 3:5. A particularly preferred solid sugar polyester is the octaester of sucrose in which there are about 7 behenic fatty acid moieties and about 1 oleic acid moiety in the molecule. Other materials include cottonseed oil or soybean oil fatty acid esters of sucrose.
(9) Organopolysiloxane oils. The organopolysiloxane oil may be volatile, non-volatile, or a mixture of volatile and non-volatile silicones. The term "nonvolatile" as used in this context refers to those silicones that are liquid under ambient conditions. The term "volatile" as used in this context refers to all other silicone oils. Suitable organopolysiloxanes can be selected from a wide variety of silicones spanning a broad range of volatilities and viscosities. Nonvolatile polysiloxanes are preferred. Examples of suitable organopolysiloxane oils include polyalkylsiloxanes, cyclic polyalkylsiloxanes, and polyalkylarylsiloxanes.
   Preferred for use herein are organopolysiloxanes selected from the group consisting of polyalkylsiloxanes, alkyl substituted dimethicones, cyclomethicones, trimethylsiloxysilicates, dimethiconols, polyalkylaryl siloxanes, and mixtures thereof. More preferred for use herein are polyalkylsiloxanes and cyclomethicones. Preferred among the polyalkylsiloxanes are dimethicones, as described in US 5,968,528.
(10) Vegetable oils and hydrogenated vegetable oils. Examples of vegetable oils and hydrogenated vegetable oils include safflower oil, castor oil, coconut oil, cottonseed oil, menhaden oil, palm kernel oil, palm oil, peanut oil, soybean oil, rapeseed oil, linseed oil, rice bran oil, pine oil, sesame oil, sunflower seed oil, hydrogenated safflower oil, hydrogenated castor oil, hydrogenated coconut oil, hydrogenated cottonseed oil, hydrogenated menhaden oil, hydrogenated palm kernel oil, hydrogenated palm oil, hydrogenated peanut oil, hydrogenated soybean oil, hydrogenated rapeseed oil, hydrogenated linseed oil, hydrogenated rice bran oil, hydrogenated sesame oil, hydrogenated sunflower seed oil, and mixtures thereof.
(11) animal fats and oils e.g., lanolin and derivatives thereof, cod liver oil.
(12) Also useful are C4-C20 alkyl ethers of polypropylene glycols, C1-C20 carboxylic acid esters of polypropylene glycols, and di-C8-C30 alkyl ethers. Nonlimiting examples of these materials include PPG-14 butyl ether, PPG-15 stearyl ether, dioctyl ether, dodecyl octyl ether, and mixtures thereof.

### b) Hydrophilic component

Emulsions of the present invention also comprise a hydrophilic component, e.g., water or other hydrophilic diluent. The hydrophilic phase can thus comprise water, or a combination of water and one or more water soluble or dispersible ingredients. Hydrophilic components comprising water are preferred.

### (c) Other components

Emulsions and other topical compositions of the present invention may comprise a variety of other ingredients such as disclosed herein. As will be understood by the skilled artisan, a given component will distribute primarily into either a hydrophilic phase or hydrophobic phase, depending on the hydrophilicity of the component in the composition.

Emulsions of the present invention preferably include one or more compounds selected from emulsifiers, surfactants, structuring agents, and thickeners.

### (1) Emulsifiers/Surfactants

The emulsion may contain an emulsifier and/or surfactant, generally to help disperse and suspend the discontinuous phase within the continuous phase. A wide variety of such agents can be employed. Known or conventional emulsifiers/surfactants can be used in the composition, provided that the selected agent is chemically and physically compatible with essential components of the composition, and provides the desired dispersion characteristics. Suitable agents include non-silicone-containing emulsifiers/surfactants, silicone emulsifiers/surfactants, and mixtures thereof.

In a preferred embodiment, the composition comprises a hydrophilic emulsifier or surfactant. The compositions of the present invention preferably comprise from about 0.05% to about 5%, more preferably from about 0.05% to about 1% of at least one hydrophilic surfactant.

Preferred hydrophilic surfactants are selected from nonionic surfactants. Among the nonionic surfactants that are useful herein are those that can be broadly defined as condensation products of long chain alcohols, e.g. C8-30 alcohols, with sugar or starch polymers, i.e., glycosides. Examples of long chain alcohols from which the alkyl group can be derived include decyl alcohol, cetyl alcohol, stearyl alcohol, lauryl alcohol, myristyl alcohol, oleyl alcohol, and the like. Commercially available examples of these surfactants include decyl polyglucoside (available as APG 325 CS from Henkel) and lauryl polyglucoside (available as APG 600 CS and 625 CS from Henkel).

Other useful nonionic surfactants include the condensation products of alkylene oxides with fatty acids (i.e. alkylene oxide esters of fatty acids). Other nonionic surfactants are the condensation products of alkylene oxides with fatty alcohols (i.e. alkylene oxide ethers of fatty alcohols). Still other nonionic surfactants are the condensation products of alkylene oxides with both fatty acids and fatty alcohols [i.e. wherein the polyalkylene oxide portion is esterified on one end with a fatty acid and etherified (i.e. connected via an ether linkage) on the other end with a fatty alcohol]. Nonlimiting examples of these alkylene oxide derived nonionic surfactants include ceteth-6, ceteth-10, ceteth-12, ceteareth-6, ceteareth-10, ceteareth-12, steareth-6, steareth-10, steareth-12, PEG-6 stearate, PEG-10 stearate, PEG-100 stearate, PEG-12 stearate, PEG-20 glyceryl stearate, PEG-80 glyceryl tallowate, PEG-10 glyceryl stearate, PEG-30 glyceryl cocoate, PEG-80 glyceryl cocoate, PEG-200 glyceryl tallowate, PEG-8 dilaurate, PEG-10 distearate, and mixtures thereof.

Still other useful nonionic surfactants include polyhydroxy fatty acid amide surfactants.

Preferred among the nonionic surfactants are those selected from the group consisting of steareth-21, ceteareth-20, ceteareth-12, sucrose cocoate, steareth-100, PEG-100 stearate, and mixtures thereof.

Other nonionic surfactants suitable for use herein include sugar esters and polyesters, alkoxylated sugar esters and polyesters, C1-C30 fatty acid esters of C1-C30 fatty alcohols, alkoxylated derivatives of C1-C30 fatty acid esters of C1-C30 fatty alcohols, alkoxylated ethers of C1-C30 fatty alcohols, polyglyceryl esters of C1-C30 fatty acids, C1-C30 esters of polyols, C1-30 ethers of polyols, alkyl phosphates, polyoxyalkylene fatty ether phosphates, fatty acid amides, acyl lactylates, and mixtures thereof. Nonlimiting examples of these non-silicon-containing emulsifiers include: polyethylene glycol 20 sorbitan monolaurate (Polysorbate 20), polyethylene glycol 5 soya sterol, Steareth-20, Ceteareth-20, PPG-2 methyl glucose ether distearate, Ceteth-10, Polysorbate 80, cetyl phosphate, potassium cetyl phosphate, diethanolamine cetyl phosphate, Polysorbate 60, glyceryl stearate, polyoxyethylene 20 sorbitan trioleate (Polysorbate 85), sorbitan monolaurate, polyoxyethylene 4 lauryl ether sodium stearate, polyglyceryl-4 isostearate, hexyl laurate, PPG-2 methyl glucose ether distearate, PEG-100 stearate, and mixtures thereof.

Another emulsifier useful herein are fatty acid ester blends based on a mixture of sorbitan or sorbitol fatty acid ester and sucrose fatty acid ester. The preferred fatty acid ester emulsifier is a blend of sorbitan or sorbitol C₁₆-C₂₀ fatty acid ester with sucrose C₁₀-C₁₆ fatty acid ester, especially sorbitan stearate and sucrose cocoate. This is commercially available from ICI under the trade name Arlatone 2121.

The hydrophilic surfactants useful herein can alternatively or additionally include any of a wide variety of cationic, anionic, zwitterionic, and amphoteric surfactants such as are known in the art. See, e.g., McCutcheon's, Detergents and Emulsifiers, North American Edition (1986), published by Allured Publishing Corporation.

Exemplary cationic surfactants useful herein include those disclosed in U.S. Pat. No. 5,968,528. The cationic surfactants useful herein include cationic ammonium salts such as quaternary ammonium salts, and amino- amides.

A wide variety of anionic surfactants are also useful herein. See e.g., US. Pat. No. 5,968,528. Nonlimiting examples of anionic surfactants include the alkoyl isethionates (e.g., C₁₂-C₃₀), alkyl and alkyl ether sulfates and salts thereof, alkyl and alkyl ether phosphates and salts thereof, alkyl methyl taurates (e.g., C₁₂ -C₃₀), and soaps (e.g., alkali metal salts, e.g., sodium or potassium salts) of fatty acids.

Amphoteric and zwitterionic surfactants are also useful herein. Examples of amphoteric and zwitterionic surfactants which can be used in the compositions of the present invention are those which are broadly described as derivatives of aliphatic secondary and tertiary amines in which the aliphatic radical can be straight or branched chain and wherein one of the aliphatic substituents contains from about 8 to about 22 carbon atoms (preferably C₈ -C₁₈) and one contains an anionic water solubilizing group, e.g., carboxy, sulfonate, sulfate, phosphate, or phosphonate. Examples are alkyl imino acetates, and iminodialkanoates and aminoalkanoates, imidazolinium and ammonium derivatives. Other suitable amphoteric and zwitterionic surfactants are those selected from the group consisting of betaines, sultaines, hydroxysultaines, alkyl sarcosinates (e.g., C₁₂-C₃₀), and alkanoyl sarcosinates.

Preferred emulsions of the present invention include a silicone containing emulsifier or surfactant. A wide variety of silicone emulsifiers are useful herein. These silicone emulsifiers are typically organically modified organopolysiloxanes, also known to those skilled in the art as silicone surfactants. Useful silicone emulsifiers include dimethicone copolyols. These materials are polydimethyl siloxanes which have been modified to include polyether side chains such as polyethylene oxide chains, polypropylene oxide chains, mixtures of these chains, and polyether chains containing moieties derived from both ethylene oxide and propylene oxide. Other examples include alkyl-modified dimethicone copolyols, i.e., compounds which contain C2-C30 pendant side chains. Still other useful dimethicone copolyols include materials having various cationic, anionic, amphoteric, and zwitterionic pendant moieties.

Nonlimiting examples of dimethicone copolyols and other silicone surfactants useful as emulsifiers herein include polydimethylsiloxane polyether copolymers with pendant polyethylene oxide sidechains, polydimethylsiloxane polyether copolymers with pendant polypropylene oxide sidechains, polydimethylsiloxane polyether copolymers with pendant mixed polyethylene oxide and polypropylene oxide sidechains, polydimethylsiloxane polyether copolymers with pendant mixed poly(ethylene)(propylene)oxide sidechains, polydimethylsiloxane polyether copolymers with pendant organobetaine sidechains, polydimethylsiloxane polyether copolymers with pendant carboxylate sidechains, polydimethylsiloxane polyether copolymers with pendant quaternary ammonium sidechains; and also further modifications of the preceding copolymers containing pendant C2-C30 straight, branched, or cyclic alkyl moieties. Examples of commercially available dimethicone copolyols useful herein sold by Dow Corning Corporation are Dow Corning@ 190, 193, Q2-5220, 2501 Wax, 2-5324 fluid, and 3225C (this later material being sold as a mixture with cyclomethicone). Cetyl dimethicone copolyol is commercially available as a mixture with polyglyceryl-4 isostearate (and) hexyl laurate and is sold under the tradename ABIL® WE-09 (available from Goldschmidt). Cetyl dimethicone copolyol is also commercially available as a mixture with hexyl laurate (and) polyglyceryl-3 oleate (and) cetyl dimethicone and is sold under the tradename ABIL® WS-08 (also available from Goldschmidt). Other nonlimiting examples of dimethicone copolyols also include lauryl dimethicone copolyol, dimethicone copolyol acetate, dimethicone copolyol adipate, dimethicone copolyolamine, dimethicone copolyol behenate, dimethicone copolyol butyl ether, dimethicone copolyol hydroxy stearate, dimethicone copolyol isostearate, dimethicone copolyol laurate, dimethicone copolyol methyl ether, dimethicone copolyol phosphate, and dimethicone copolyol stearate.

Dimethicone copolyol emulsifiers useful herein are described, for example, in U.S. Pat. No. 5,968,528.

### (2) Structuring Agent

The compositions hereof, and especially the emulsions hereof, may contain a structuring agent. Structuring agents are particularly preferred in the oil-in-water emulsions of the present invention. Without being limited by theory, it is believed that the structuring agent assists in providing rheological characteristics to the composition which contribute to the stability of the composition. For example, the structuring agent tends to assist in the formation of the liquid crystalline gel network structures. The structuring agent may also function as an emulsifier or surfactant. Preferred compositions of this invention comprise from about 1% to about 20%, more preferably from about 1% to about 10%, most preferably from about 2% to about 9%, of one or more structuring agents.

Preferred structuring agents are those having an HLB of from about 1 to about 8 and having a melting point of at least about 45 C°. Suitable structuring agents are those selected from the group consisting of saturated C14 to C30 fatty alcohols, saturated C16 to C30 fatty alcohols containing from about 1 to about 5 moles of ethylene oxide, saturated C16 to C30 diols, saturated C16 to C30 monoglycerol ethers, saturated C16 to C30 hydroxy fatty acids, C14 to C30 hydroxylated and nonhydroxylated saturated fatty acids, C14 to C30 saturated ethoxylated fatty acids, amines and alcohols containing from about 1 to about 5 moles of ethylene oxide diols, C14 to C30 saturated glyceryl mono esters with a monoglyceride content of at least 40%, C14 to C30 saturated polyglycerol esters having from about 1 to about 3 alkyl group and from about 2 to about 3 saturated glycerol units, C14 to C30 glyceryl mono ethers, C14 to C30 sorbitan mono/diesters, C14 to C30 saturated ethoxylated sorbitan mono/diesters with about 1 to about 5 moles of ethylene oxide, C14 to C30 saturated methyl glucoside esters, C14 to C30 saturated sucrose mono/diesters, C14 to C30 saturated ethoxylated methyl glucoside esters with about 1 to about 5 moles of ethylene oxide, C14 to C30 saturated polyglucosides having an average of between 1 to 2 glucose units and mixtures thereof, having a melting point of at least about 45C°.

The preferred structuring agents of the present invention are selected from the group consisting of stearic acid, palmitic acid, stearyl alcohol, cetyl alcohol, behenyl alcohol, stearic acid, palmitic acid, the polyethylene glycol ether of stearyl alcohol having an average of about 1 to about 5 ethylene oxide units, the polyethylene glycol ether of cetyl alcohol having an average of about 1 to about 5 ethylene oxide units, and mixtures thereof. More preferred structuring agents of the present invention are selected from the group consisting of stearyl alcohol, cetyl alcohol, behenyl alcohol, the polyethylene glycol ether of stearyl alcohol having an average of about 2 ethylene oxide units (steareth-2), the polyethylene glycol ether of cetyl alcohol having an average of about 2 ethylene oxide units, and mixtures thereof. Even more preferred structuring agents are selected from the group consisting of stearic acid, palmitic acid, stearyl alcohol, cetyl alcohol, behenyl alcohol, steareth-2, and mixtures thereof.

### (3) Thickening Agent (Including Thickeners and Gelling Agents)

The compositions of the present invention can also comprise a thickening agent, preferably from about 0.1% to about 5%, more preferably from about 0.1% to about 3%, and most preferably from about 0.25% to about 2%, of a thickening agent.

Nonlimiting classes of thickening agents include those selected from the group consisting of:

### (i) Carboxylic Acid Polymers

These polymers are crosslinked compounds containing one or more monomers derived from acrylic acid, substituted acrylic acids, and salts and esters of these acrylic acids and the substituted acrylic acids, wherein the crosslinking agent contains two or more carbon--carbon double bonds and is derived from a polyhydric alcohol. The preferred carboxylic acid polymers are of two general types. The first type of polymer is a crosslinked homopolymer of an acrylic acid monomer or derivative thereof (e.g., wherein the acrylic acid has substituents on the two and three carbon positions independently selected from the group consisting of C1-4 alkyl, --CN, --COOH, and mixtures thereof). The second type of polymer is a crosslinked copolymer having a first monomer selected from the group consisting of an acrylic acid monomer or derivative thereof (as just described in the previous sentence), a short chain alcohol (i.e., a C1-4) acrylate ester monomer or derivative thereof (e.g., wherein the acrylic acid portion of the ester has substituents on the two and three carbon positions independently selected from the group consisting of C1-4 alkyl, --CN, --COOH, and mixtures thereof), and mixtures thereof; and a second monomer which is a long chain alcohol (i.e. C8-40) acrylate ester monomer or derivative thereof (e.g., wherein the acrylic acid portion of the ester has substituents on the two and three carbon positions independently selected from the group consisting of C1-4 alkyl, --CN, --COOH, and mixtures thereof). Combinations of these two types of polymers are also useful herein.
In the first type of crosslinked homopolymers, the monomers are preferably selected from the group consisting of acrylic acid, methacrylic acid, ethacrylic acid, and mixtures thereof, with acrylic acid being most preferred. In the second type of crosslinked copolymers the acrylic acid monomer or derivative thereof is preferably selected from the group consisting of acrylic acid, methacrylic acid, ethacrylic acid, and mixtures thereof, with acrylic acid, methacrylic acid, and mixtures thereof being most preferred. The short chain alcohol acrylate ester monomer or derivative thereof is preferably selected from the group consisting of C1-4 alcohol acrylate esters, C1-4 alcohol methacrylate esters, C1-4 alcohol ethacrylate esters, and mixtures thereof, with the C1-4 alcohol acrylate esters, C1-4 alcohol methacrylate esters, and mixtures thereof, being most preferred. The long chain alcohol acrylate ester monomer is selected from C8-40 alkyl acrylate esters, with C10-30 alkyl acrylate esters being preferred.
The crosslinking agent in both of these types of polymers is a polyalkenyl polyether of a polyhydric alcohol containing more than one alkenyl ether group per molecule, wherein the parent polyhydric alcohol contains at least 3 carbon atoms and at least 3 hydroxyl groups. Preferred crosslinkers are those selected from the group consisting of allyl ethers of sucrose and allyl ethers of pentaerythritol, and mixtures thereof.
Examples of commercially available homopolymers of the first type useful herein include the carbomers, which are homopolymers of acrylic acid crosslinked with allyl ethers of sucrose or pentaerytritol. The carbomers are available as the Carbopol® 900 series from B.F. Goodrich (e.g., Carbopol® 954). Examples of commercially available copolymers of the second type useful herein include copolymers of C10-30 alkyl acrylates with one or more monomers of acrylic acid, methacrylic acid, or one of their short chain (i.e. C1-4 alcohol) esters, wherein the crosslinking agent is an ally ether of sucrose or pentaerytritol. These copolymers are known as acrylates/C10-30 alkyl acrylate crosspolymers and are commercially available as Carbopol® 1342, Carbopol® 1382Pemulen TR-1, and Pemulen TR-2, from B. F. Goodrich. In other words, examples of carboxylic acid polymer thickeners useful herein are those selected from the group consisting of carbomers, acrylates/C10-C30 alkyl acrylate crosspolymers, and mixtures thereof.

### (ii) Crosslinked Polyacrylate Polymers

The crosslinked polyacrylate polymers useful as thickeners or gelling agents include both cationic and nonionic polymers, with the cationics being generally preferred. Non-limiting examples of suitable crosslinked polyacrylate polymers are disclosed in US 5,968,528.

### (iii) Polyacrylamide Polymers

Also useful herein are polyacrylamide polymers, especially non-ionic polyacrylamide polymers including substituted branched or unbranched polymers. These polymers can be formed from a variety of monomers including acrylamide and methacrylamide which are unsubstituted or substituted with one or two alkyl groups (preferably C1 to C5). Preferred are acrylate amide and methacrylate amide monomers in which the amide nitrogen is unsubstituted, or substituted with one or two C1 to C5 alkyl groups (preferably methyl, ethyl, or propyl), for example, acrylamide, methacrylamide, N-methacrylamide, N-methylmethacrylamide, N,N-dimethylmethacrylamide, N-isopropylacrylamide, N-isopropylmethacrylamide, and N,N-dimethylacrylamide. These polymers have a molecular weight greater than about 1,000,000 preferably greater than about 1,5000,000 and range up to about 30,000,000. Most preferred among these polyacrylamide polymers is the non-ionic polymer given the CTFA designation polyacrylamide and isoparaffin and laureth-7, available under the Tradename Sepigel 305 from Seppic Corporation (Fairfield, N.J.).
Other polyacrylamide polymers useful herein include multi-block copolymers of acrylamides and substituted acrylamides with acrylic acids and substituted acrylic acids. Commercially available examples of these multi-block copolymers include Hypan SR150H, SS500V, SS500W, SSSA100H, from Lipo Chemicals, Inc., (Patterson, N.J.).

### (iv) Polysaccharides

A wide variety of polysaccharides are useful herein. By "polysaccharides" are meant gelling agents containing a backbone of repeating sugar (i.e. carbohydrate) units. Nonlimiting examples of polysaccharide gelling agents include those selected from the group consisting of cellulose, carboxymethyl hydroxyethylcellulose, cellulose acetate propionate carboxylate, hydroxyethylcellulose, hydroxyethyl ethylcellulose, hydroxypropylcellulose, hydroxypropyl methylcellulose, methyl hydroxyethylcellulose, microcrystalline cellulose, sodium cellulose sulfate, and mixtures thereof. Also useful herein are the alkyl substituted celluloses. In these polymers, the hydroxy groups of the cellulose polymer is hydroxyalkylated (preferably hydroxyethylated or hydroxypropylated) to form a hydroxyalkylated cellulose which is then further modified with a C10-C30 straight chain or branched chain alkyl group through an ether linkage, Typically these polymers are ethers of C10-C30 straight or branched chain alcohols with hydroxyalkylcelluloses. Examples of alkyl groups useful herein include those selected from the group consisting of stearyl, isostearyl, lauryl, myristyl, cetyl, isocetyl, cocoyl (i.e. alkyl groups derived from the alcohols of coconut oil), palmityl, oleyl, linoleyl, linolenyl, ricinoleyl, behenyl, and mixtures thereof. Preferred among the alkyl hydroxyalkyl cellulose ethers is the material given the CTFA designation cetyl hydroxyethylcellulose, which is the ether of cetyl alcohol and hydroxyethylcellulose. This material is sold under the tradename Natrosol® CS Plus from Aqualon Corporation.

### v) Gums

Other additional thickening and gelling agents useful herein include materials which are primarily derived from natural sources. Nonlimiting examples of these gelling agent gums include materials selected from the group consisting of acacia, agar, algin, alginic acid, ammonium alginate, amylopectin, calcium alginate, calcium carrageenan, carnitine, carrageenan, dextrin, gelatin, gellan gum, guar gum, guar hydroxypropyltrimonium chloride, hectorite, hyaluroinic acid, hydrated silica, hydroxypropyl chitosan, hydroxypropyl guar, karaya gum, kelp, locust bean gum, natto gum, potassium alginate, potassium carrageenan, propylene glycol alginate, sclerotium gum, sodium carboyxmethyl dextran, sodium carrageenan, tragacanth gum, xanthan gum, and mixtures thereof.

### (vi) Crosslinked Vinyl Ether/Maleic Anhydride Copolymers

Other additional thickening and gelling agents useful herein include crosslinked copolymers of alkyl vinyl ethers and maleic anhydride.

### (vii) Crosslinked Poly(N-vinylpyrrolidones)

Crosslinked polyvinyl(N-pyrrolidones) useful herein as additional thickening and gelling agents These gelling agents typically contain from about 0.25% to about 1% by weight of a crosslinking agent selected from the group consisting of divinyl ethers and diallyl ethers of terminal diols containing from about 2 to about 12 carbon atoms, divinyl ethers and diallyl ethers of polyethylene glycols containing from about 2 to about 600 units, dienes having from about 6 to about 20 carbon atoms, divinyl benzene, vinyl and allyl ethers of pentaerythritol, and the like.

Preferred compositions of the present invention include a thickening agent selected from the group consisting of carboxylic acid polymers, crosslinked polyacrylate polymers, polyacrylamide polymers, and mixtures thereof, more preferably selected from the group consisting of crosslinked polyacrylate polymers, polyacrylamide polymers, and mixtures thereof.

### Optional Components

The topical compositions of the present invention may comprise a wide variety of optional components, provided that such optional components are physically and chemically compatible with the essential components described herein, and do not unduly impair stability, efficacy or other use benefits associated with the compositions of the present invention. Optional components may be dispersed, dissolved or the like in the carrier of the present compositions.

Optional components include aesthetic agents and active agents. For example, the compositions may include, in addition to the essential components of the invention, absorbents (including oil absorbents such as clays an polymeric absorbents), abrasives, anticaking agents, antifoaming agents, antimicrobial agents (e.g., a compound capable of destroying microbes, preventing the development of microbes or preventing the pathogenic action of microbes and useful, for example, in controlling acne and/or preserving the topical composition), binders, biological additives, buffering agents, bulking agents, chemical additives, cosmetic biocides, denaturants, cosmetic astringents, drug astringents, external analgesics, film formers, humectants, opacifying agents, fragrances, perfumes, pigments, colorings, essential oils, skin sensates, emollients, skin soothing agents, skin healing agents, pH adjusters, plasticizers, preservatives, preservative enhancers, propellants, reducing agents, skin-conditioning agents, skin penetration enhancing agents, skin protectants, solvents, suspending agents, emulsifiers, thickening agents, solubilizing agents, polymers for aiding the film-forming properties and substantivity of the composition (such as a copolymer of eicosene and vinyl pyrrolidone, an example of which is available from GAF Chemical Corporation as Ganex® V-220), waxes, sunscreens, sunblocks, ultraviolet light absorbers or scattering agents, sunless tanning agents, antioxidants and/or radical scavengers, chelating agents, sequestrants, anti-acne agents, anti-inflammatory agents, anti-androgens, depilation agents, desquamation agents/exfoliants, organic hydroxy acids, vitamins and derivatives thereof (including water dispersible or soluble vitamins such as Vitamin C and ascorbyl phosphates), compounds which stimulate collagen production, and natural extracts. Such other materials are known in the art. Nonexclusive examples of such materials are described in Pharmaceutical Dosage Forms-Disperse Systems; Lieberman, Rieger & Banker, Vols. 1 (1988) & 2 (1989); or in US 5,968,528.

The composition of the invention may comprise an emollient. The emollient may be selected from one or more of the following classes: Triglyceride esters which include, but are not limited to, vegetable and animal fats and oils such as castor oil, cocoa butter, safflower oil, cottonseed oil, corn oil, olive oil, cod liver oil, almond oil, avocado oil, palm oil, sesame oil, squalene, kikui oil and soybean oil; Acetoglyceride esters, such as acetylated monoglycerides; Ethoxylated glycerides, such as ethoxylated glyceryl monostearate; Alkyl esters of fatty acids having 10 to 20 carbon atoms which include, but are not limited to, methyl; isopropyl, and butyl esters of fatty acids such as hexyl laurate, isohexyl laurate, isohexyl palmitate, isopropyl palmitate, methyl palmitate, decyloleate, isodecyl oleate, hexadecyl stearate decyl stearate, isopropyl isostearate, methyl isostearate, diisopropyl adipate, diisohexyl adipate, dihexyldecyl adipate, diisopropyl sebacate, lauryl lactate, myristyl lactate, and cetyl lactate; Alkenyl esters of fatty acids having 10 to 20 carbon atoms such as oleyl myristate, oleyl stearate, and oleyl oleate; Fatty acids having 10 to 20 carbon atoms such as pelargonic, lauric, myristic, palmitic, stearic, isostearic, hydroxystearic, oleic, linoleic, ricinoleic, arachidic, behenic, and erucic acids; Fatty alcohols having 10 to 20 carbon atoms such as lauryl, myristyl, cetyl, hexadecyl, stearyl, isostearyl, hydroxystearyl, oleyl, ricinoleyl, behenyl, erucyl, and 2-octyl dodecanyl alcohols; Lanolin and lanolin derivatives such as lanolin, lanolin oil, lanolin wax, lanolin alcohols, lanolin fatty acids, isopropyl lanolate, ethoxylated cholesterol, propoxylated lanolin alcohols, acetylated lanolin alcohols, lanolin alcohols linoleate, lanolin alcohols ricinoleate, acetate of lanolin alcohols ricinoleate, acetate of ethoxylated alcohols-esters, hydrogenolysis of lanolin, ethoxylated hydrogenated lanolin, and liquid and semisolid lanolin absorption bases; Polyhydric alcohol esters such as ethylene glycol mono and di-fatty acid esters, diethylene glycol mono-and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol polyfatty esters, ethoxylated glyceryl monostearate, 1,2-butylene glycol monostearate, 1,2-butylene glycol distearate, sorbitan fatty acid esters, and polyoxyethylene sorbitan fatty acid esters; Wax esters such as beeswax, spermaceti, myristyl myristate, stearyl stearate; Beeswax derivatives such as polyoxyethylene sorbitol beeswax which are reaction products of beeswax with ethoxylated sorbitol of varying ethylene oxide content, forming a mixture of ether esters; Vegetable waxes including, but not limited to, carnauba and candelilla waxes; Phospholipids such as lecithin and derivatives; Sterols including, but not limited to, cholesterol and cholesterol fatty acid esters; and Amides such as fatty acid amides, ethoxylated fatty acid amides, and solid fatty acid alkanolamides.

The composition may comprise humectants, e.g., of the polyhydric alcohol-type. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, erythritol, threitol, pentaerythritol, xylitol, glucitol, mannitol, hexylene glycol, butylene glycol (e.g., 1,3-butylene glycol), hexane triol (e.g., 1,2,6-hexanetriol), glycerol, ethoxylated glycerol, propoxylated glycerol, sodium 2-pyrrolidone-5-carboxylate, soluble collagen, dibutyl phthalate, gelatin and mixtures thereof.

Further optional components are guanidine; glycolic acid and glycolate salts (e.g. ammonium and quaternary alkyl ammonium); lactic acid and lactate salts (e.g. ammonium and quaternary alkyl ammonium); aloe vera in any of its variety of forms (e.g., aloe vera gel); sugar and starch derivatives (e.g., alkoxylated glucose); hyaluronic acid and derivatives thereof (e.g., salt derivatives such as sodium hyaluraonate); lactamide monoethanolamine; acetamide monoethanolamine; urea; panthenol; sugars; starches; silicone fluids; silicone gums; and mixtures thereof. Also useful are the propoxylated glycerols.

### Preferred embodiments:

Both hydrophilic and lipophilic creams were investigated. These creams are made up of highly biocompatible ingredients. In particular, the lipid phases of these cream systems may be made up of any vegetal oil, such as olive oil, sweet almond oil, avocado oil, jojoba oil and others, of mineral oils, such as vaseline or others, and/or of any synthetic oil phase, such as esters of fatty acids, short, medium and long chain triglycerides. To prepare these systems dermatologically acceptable surfactants that are able to provide a certain physicochemical stability to creams may be used, e.g. lecithin, tween 80 and the like. Gel-based formulations were also investigated as potential formulations for the topical application of valproic acid, its salts and derivatives. Gels can be prepared by using any viscosizing agent that is able to provide the desired rheological features to formulations. Also in this case, agents to be used for the preparation of gel-based formulations must have a certain human skin tolerability, i.e. sepigel, zilgel, carbopol, xantan gum, gelatin, PVP, PEO and the like.

### Dosing

Specific topical dose levels for any particular patient may be employed depending upon a variety of factors including the age, body weight, general health, sex, diet, and prior medication, and the severity of the particular disease of the patient, and the activity of specific compounds employed, time of administration, rate of excretion, the duration of the treatment, other drugs, compounds, and/or materials used in combination. It will be appreciated that the appropriate dosage of the active compounds, and compositions comprising the active compounds, can vary from patient to patient. Determining the optimal dosage will generally involve balancing of the level of therapeutic benefit against any risk or deleterious side effects of the treatments of the present invention.

Topical administration in vivo can be effected in one dose, continuously or intermittently throughout the course of treatment. Methods of-determining the most effective means and dosages of administration are well known to those of skill in the art and will vary with the formulation used for therapy, the purpose of the therapy, the target cell being treated, and the subject being treated. Single or multiple administrations can be carried out with the dose level and pattern being selected by the treating physician.

In general, a suitable dose of the active compound, i.e., VPA or a VPA derivative, comprises a concentration of at least about 0.1 % by weight, preferably 0.1 % to 6% per weight, more preferably 0.3% to 5%, more preferably 0.5% to 4% and even more preferably 1% to 4% per weight and the active ingredient is suspended in a pharmaceutically acceptable carrier. Where the active ingredient is a salt, an ester, prodrug, or the like, the amount administered is calculated on the basis the parent compound and so the actual weight to be used is increased proportionately.

If the active compound, i.e., VPA or a VPA derivative, is used in combination with retinoids, a suitable dose of this active compound is in the range of at least 0.1% by weight, preferably 0.1 % to 6% per weight, more preferably 0.3% to 5%, more preferably 0.5% to 4% and even more preferably 1% to 4% per weight and for the retinoid used a suitable dose of the active compound is in the range of about 0.01% to about 1% by weight, preferably 0.05% to 0.5% per weight.

If the active compound, i.e., VPA or a VPA derivative, is used in combination with 5-Fluorouracil, a suitable dose of the active compound is in the range of at least 0.1% by weight, preferably 0.1 % to 6% per weight, more preferably 0.3% to 5%, more preferably 0.5% to 4% and even more preferably 1% to 4% per weight and for 5-Fluorouracil a suitable dose of the active compound is in the range of about 0.1% to about 10% by weight, preferably 1 % to 10% per weight.

In a preferred embodiment of this invention, VPA is used topically applied once to three times daily at an accumulated total daily dose of 0.5 mg (milligram) to 10 mg (milligram) per lesion of approximately 1 cm² (qcm). In a more preferred embodiment, VPA is used topically applied once to three times daily at an accumulated total daily dose of 1 mg (milligram) to 8 mg (milligram) per lesion of approximately 1 cm² (qcm). in an even more preferred embodiment, VPA is used topically applied once to three times daily at an accumulated total daily dose of 2 mg (milligram) to 6 mg (milligram) per lesion of approximately 1 cm² (qcm).

The actual daily dose depends on the size of the lesion treated, and accordingly increases with increased lesion size, including lesion sizes of up to approximately 100 cm² (qcm) with according increases of daily doses.

In other preferred embodiments of the invention the topical formulation containing VPA is made up by one of the three following formulations containing the listed ingredients (content is presented as % [w/w]):

| Formulation example 1: | |
|---|---|
| White mineral oil | 20 |
| Cetyl Alcohol | 24 |
| Cetomacrogol 1000 | 6 |
| Valproic Acid | >0.1, e.g., 0.1 to 6 |
| White vaseline | add to 100 |

| Formulation example 2: | |
|---|---|
| Zinc Oxide | 25 |
| Starch | 25 |
| Valproic Acid | >0.1, e.g., 0.1 to 6 |
| White vaseline | add to 100 |

| Formulation example 3: | |
|---|---|
| White vaseline | 25 |
| Cetyl Alcohol | 10 |
| Tween 60 | 5 |
| Glycerin | 10 |
| EDTA | 0.2 |
| Parfume (optionally) | 2 drops |
| Sodium Valproate | >0.1, e.g., 0.1 to 6 |
| Bidistilled Water | add to 100 |

### Figures

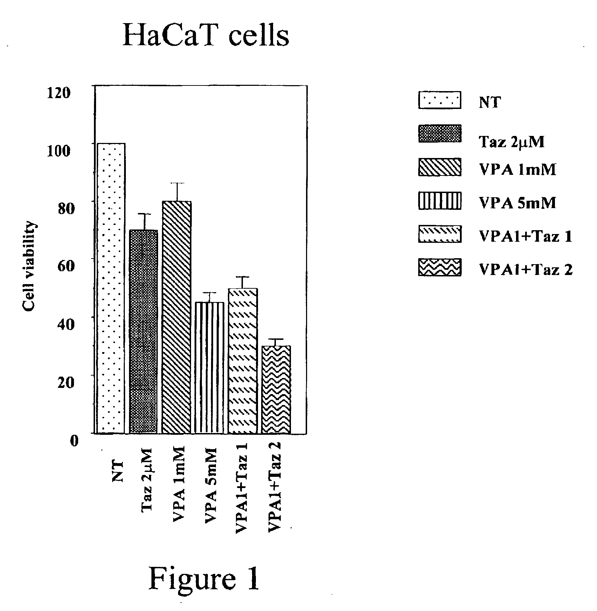

**Figure 1:** *VPA potentiates Tazarotene (Taz) induced cell growth inhibition.*
   Figure 1 depicts the results of growth assays performed on human immortalized keratinocytes (HaCaT cells), demonstrating that VPA used alone and Tazarotene, a Retinoic Acid derivative, used alone inhibit HaCaT cell growth and that the use of Tazarotene in combination with the histone deacetylase inhibitor VPA is even more effective at inhibiting cell growth than each drug used alone (NT: no treatment, Taz: Tazarotene, VPA1+Taz1: VPA 1 mM + Tazarotene 1µM, VPA1+Taz2: VPA 1mM + Tazarotene 2 µM).
**Figure 2:** *VPA potentiates Retinoic Acid (RA) induced cell growth inhibition.*

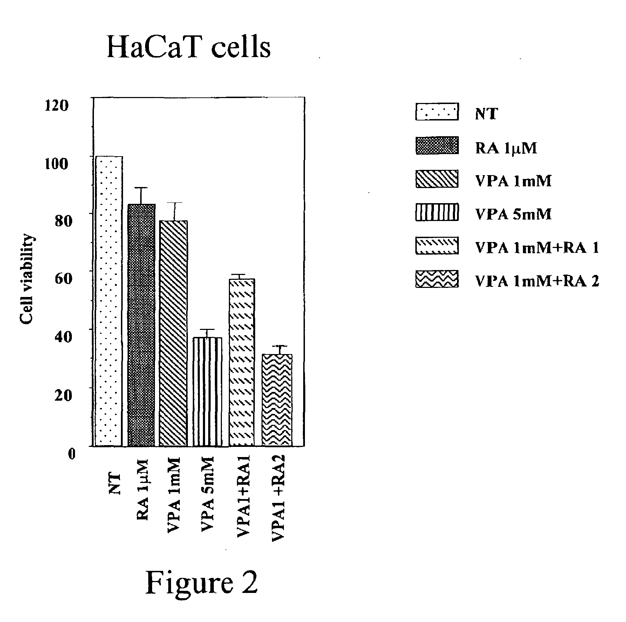

   Figure 2 shows that VPA and RA used alone inhibit growth of human immortalized keratinocytes (HaCaT cells) and that VPA potentiates Retinoic Acid induced cell growth inhibition in these cells when both drugs are used in combination (NT: no treatment, RA: Retinoic Acid, VPA1+RA1: VPA 1mM + Retinoic Acid 1µM, VPA1+RA2: VPA 1mM + Retinoic Acid 2 µM).

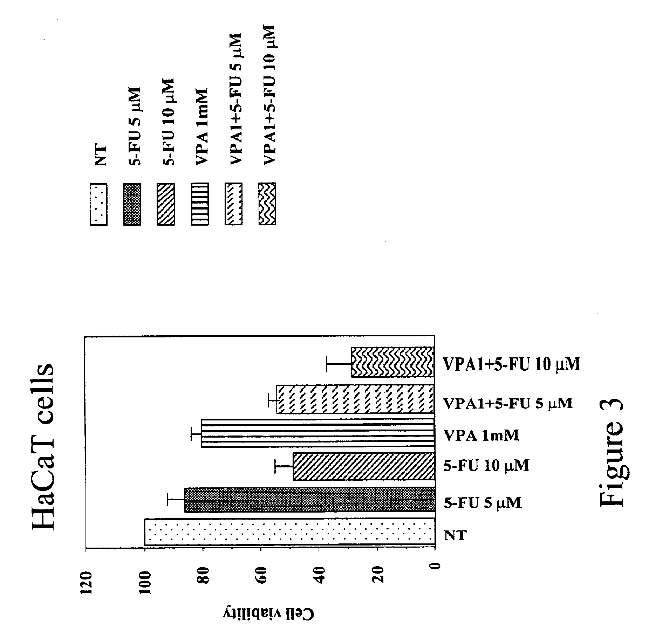

**Figure 3:** *VPA potentiates 5-Fluorouracil (5-FU) induced cell growth inhibition.*
   Figure 3 shows that VPA and 5-FU alone inhibit growth of HaCaT cells and that combinations of 5-FU (used at the concentrations indicated) plus VPA are even more effective at inhibiting HaCaT cell growth than each drug used alone (NT: no treatment, 5-FU: 5-Fluorouracil, VPA is used in combination with 5-FU at a concentration of 1mM).

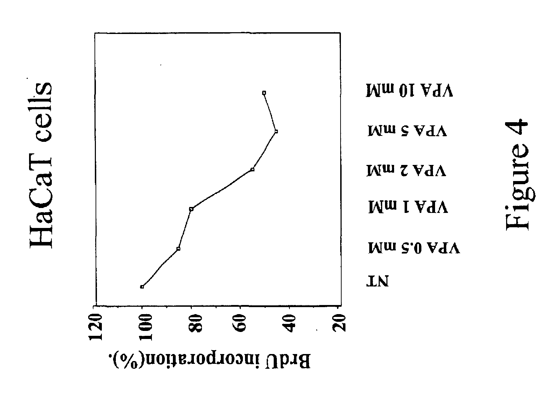

**Figure 4:** *VPA induces cell cycle arrest of human keratinocytes.*
   Figure 4 indicates that VPA alone induces cell cycle arrest in human immortalized keratinocytes (HaCaTcells).

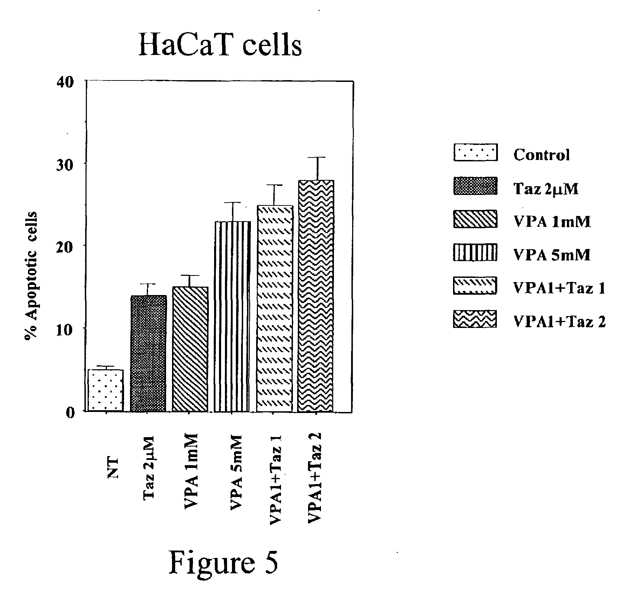

**Figure 5:** *VPA potentiates Tazarotene induced apoptosis.*
   Figure 5 demonstrates that Tazarotene and VPA used alone induces apoptosis of HaCaT cells and that combinations of Tazarotene plus VPA are even more effective at inducing apoptosis in these cells, than each drug used alone (NT: no treatment, Taz: Tazarotene, VPA1+Taz1: VPA 1mM + Tazarotene 1µM, VPA1+Taz2: VPA 1mM + Tazarotene 2 µM).

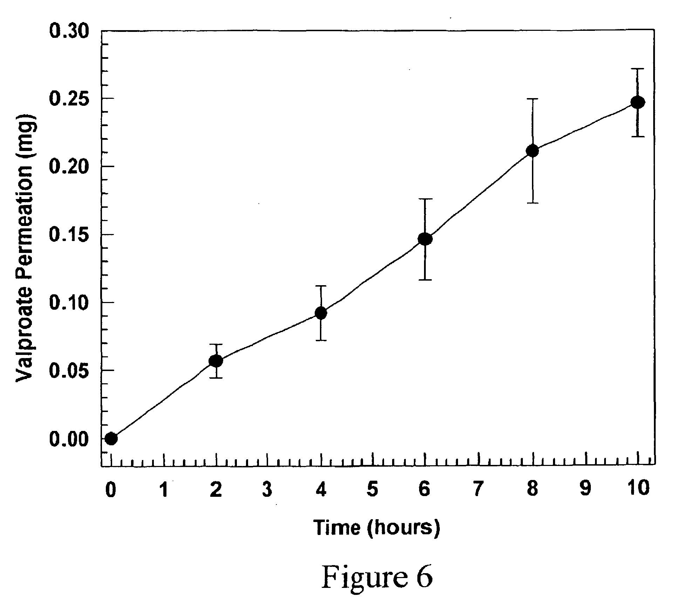

**Figure 6:** *VPA topically applied at 3% (w*/*w) permeates human skin.*
   Figure 6 shows the time dependent manner of permeation of human skin with VPA topically applied at 3% (w/w).

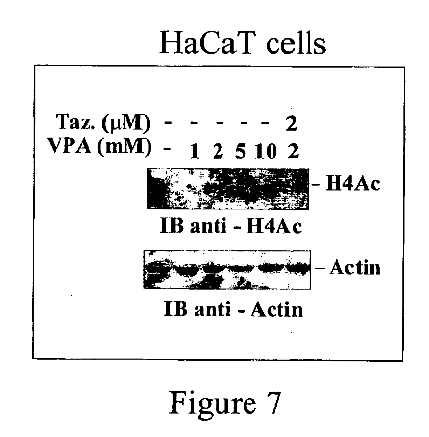

**Figure 7:** *VPA induces histone H4 acetylation in keratinocytes.*
   Figure 7 shows the VPA-induced histone H4 acetylation in HaCaT cells in a dose dependent manner.

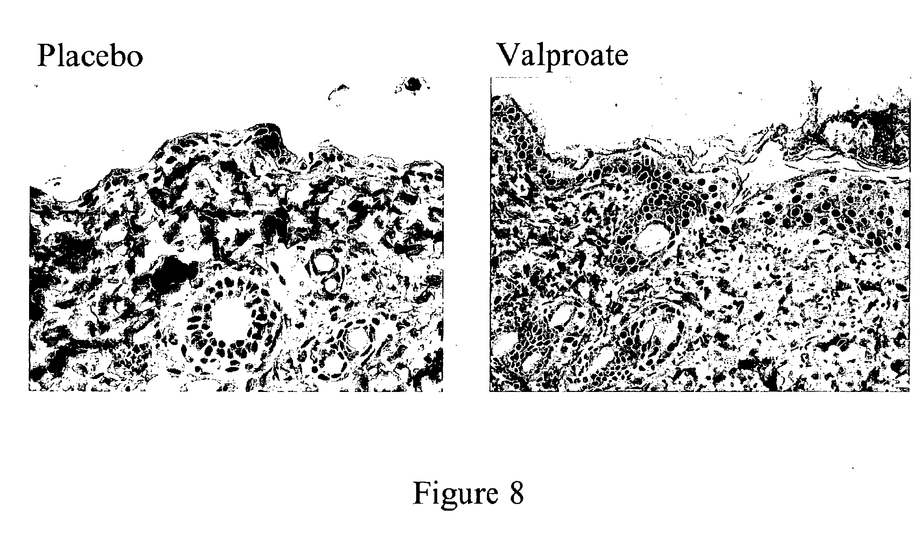

**Figure 8:** *VPA topically applied at* 3% *(w*/*w) induces skin cell acetylation in mice.*
   Figure 8 depicts the histone H4 acetylation in skin cells of mice treated with VPA topically applied at 3% (w/w).

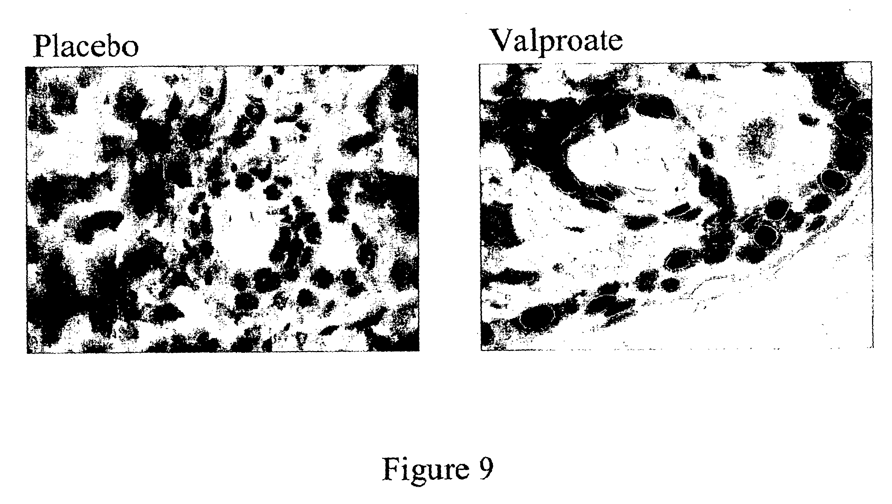

**Figure 9:** *VPA topically applied at* 3% (w/w) *induces skin cell acetylation in mice.*
   Figure 9 shows the acetylation of histone H4 in skin cells of mice induced by topical application of VPA at 3% (w/w) at a higher magnification compared to the presentation in Figure 8.

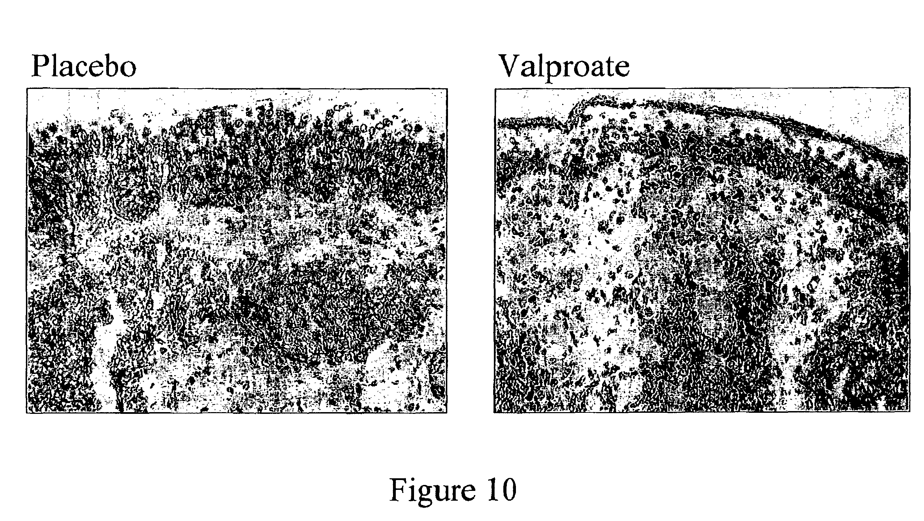

**Figure 10:** *VPA topically applied at* 3% *(w*/*w) induces acetylation of BCC nuclei.*
   Figure 10 shows the induction of acetylation of histone H4 in human Basal Cell Carcinoma (BCC) nuclei after topical application of VPA at 3% (w/w)

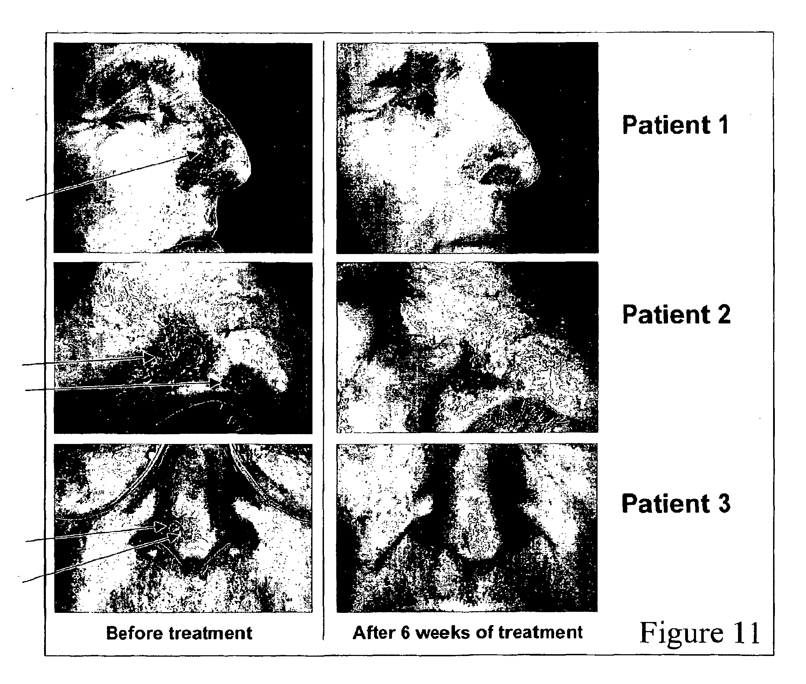

**Figure 11:** *VPA topically applied at 3% (w*/*w) potentiates the effects of Tazarotene in the treatment of superficial BCC's.*
   Figure 11 shows examples of three representative patients before treatment and after 6 weeks of combined topical treatment with Tazarotene applied at 0.1% (w/w) and with VPA applied at 3% (w/w).

### Examples

### Example 1

Synergistic cell growth inhibition and induction of apoptosis in human immortalized keratinocytes (HaCaT cells) upon treatment with VPA or the Retinoic Acid derivative Tazarotene alone and by the combination of VPA and Tazarotene (Figures 1 and 5).

### Methods:

*MTT assay*: This colorimetric assay detects the formation of formazan which is proportional to the number of cells present. Tissue culture medium containing drugs was removed and 200 µl of DME containing 2 mg/ml MTT ((3-4, 5-dimethyl thiazol-2yl) 2,5-diphenyltetrazolium bromide) was added to each well. 96-well plates were incubated at 37 °C, and 10% CO₂ for 30 minutes. Medium containing MTT was then removed and the cells were washed once with PBS. Following removal of the PBS, 200 µL DMSO was added to each well and plates were placed on a rotating platform shaker for 5 minutes. Results were obtained by reading the absorbance at a wavelength of 550 nm using a spectrophotometer. Results are expressed as percentages setting the control as 100%.

*Apoptosis assays:* Apoptosis was measured by a Beckton Dickinson FACScalibur flow cytometer by analysing the percentage of Sub-G1 DNA containing cells. 10000 events were recorded for each sample. The amplification scale was linear for all parameters. Photomultiplier tension was set as to place the peak DNA content corresponding cells in G0/G1 at channel 300 in the FL2-H vs SCC-H graphs. Cells showing a less than peak DNA content (hypodiploid cells) and high SSC-H (granular high condensed cells) were regarded as apoptotic. The experiments were repeated three times in duplicate. Error bars represent 1 standard deviation.

### Results:

Figure 1 graphically depicts the results of growth assays performed on human immortalized keratinocytes (HaCaT cells), treated for three days with the indicated compounds. Drugs were added on the same day of plating (day 0). The MTT assay (to measure cell proliferation) was carried out on day 3. Various combinations of the drugs were used in the growth assays. As can be seen, VPA and Tazarotene used alone inhibited cell growth, and the combinations of Tazarotene plus the histone deacetylase inhibitor VPA were even more effective at inhibiting cell growth than the inhibition of both drugs alone as measured by the MTT assay.
Similarly, VPA potentiates Tazarotene induced apoptosis in HaCaT cells (Figure 5). Cells were plated in 35 mm diameter wells on day 0. Drugs were added on day 1. The apoptosis assay was carried out on day 2. VPA induced a clear increase in subG1 DNA content, indicating that it induces apoptosis of human immortalized keratinocytes HaCaT. As can be seen in Figure 5, the combinations of Tazarotene plus VPA were more effective at inducing apoptosis, than the apoptosis rate seen with both drugs used alone. Thus, the combinatorial activity of these two drugs must be explained via synergistic activity of this treatment.

### Example 2

Synergistic cell growth inhibition and induction of apoptosis in human immortalized keratinocytes (HaCaT cells) upon treatment with VPA or Retinoic Acid (RA) alone and by the combination of VPA and Retinoic Acid (RA) (Figure 2).

### Method:

### MTT assay: for details see example 1

### Results:

As can be seen in Figure 2, VPA and RA used alone inhibited growth of human keratinocytes HaCaT, and VPA even potentiated RA induced cell growth inhibition when both drugs are used in combination. Cells were plated in wells on day 0. Then, drugs were added at the concentrations indicated (VPA1+RA1: VPA 1mM + Retinoic Acid 1µM, VPA1+RA2: VPA 1mM + Retinoic Acid 2 µM). The MTT assay was carried out on day 3. Various combinations of the drugs were also used in the growth assays. The combinations of RA plus the histone deacetylase inhibitor VPA were even more effective at inhibiting cell growth than each drug used alone as measured by the MTT assay. Thus, the combinatorial activity of these two drugs must be explained via synergistic activity of this treatment.

### Example 3

Synergistic cell growth inhibition and induction of apoptosis in human immortalized keratinocytes (HaCaT cells) upon treatment with VPA or 5-Fluorouracil alone and by the combination of VPA and 5-Fluorouracil (5-FU) (Figure 3).

### Method:

### MTT assay: for details see example 1

### Results:

VPA and 5-FU used alone inhibited growth of HaCaT cells, and VPA potentiated 5-FU induced growth inhibition in these cells when both drugs were used in combination (Figure 3). Drugs were added on the same day of plating (day 0). The MTT assay (to measure cell proliferation) was carried out on the third day. 5 µM or 10 µM 5-FU and 1 mM VPA were used. The combinations of 5-FU plus the HDAC inhibitor VPA were more effective at inhibiting cell growth than each drug used alone as measured by MTT assay. Thus, this combinatorial activity of these two drugs must be explained via synergistic activity of this treatment.

### Example 4

VPA induces cell cycle arrest in human immortalized keratinocytes (HaCaT cells) (Figure 4).

### Method:

*BrdU incorporation assays*: Cell proliferation was detected by labeling cells with BrdU. After 24 hours treatment with VPA at the indicated concentrations, cells were incubated with BrdU (1:100) according to the protocol of the manufacturer (ZYMED, San Francisco, CA). Cells were incubated for 1 hour in the culture medium and then fixed with 70% ethanol. The incorporated BrdU was detected by the indirect immunoperoxidase method (Amersham, Arlington Heights, IL). Briefly, the culture cells were first incubated for 1 hour with Biotin-Mouse Anti-BrdU antibody. After washing in TTBS buffer (20 mM Tris, 500 mM NaCl, 0.05% Tween-20, pH 7.5), the cells were further incubated with Biotinylated goat anti-mouse immunoglobulins for 10 min. Cells were then washed and incubated with enzyme (peroxidase) conjugate for 10 min at room temperature. Immunoreactivity was revealed by the addition of substrate-chromogen. The positive cells were counted on 10 randomly picked microscopic fields.

### Results:

HaCaT cells were plated in 35 mm diameter wells on day 0. VPA was added at the indicated concentrations on day 0. The BrdU assay was carried out on day 2. As can be seen in Figure 4, VPA produces a dose-dependent reduction of BrdU positive cells indicating that it induces cell cycle arrest and/or loss of cell viability of human keratinocytes.

### Example 5

VPA applied topically at 3% (w/w) permeates human skin in a time-dependent manner (Figure 6)

### Methods:

*Preparation of a topical applicable, aqueous VPA formulation:* A topical sodium valproate formulation was prepared by dispersing Sepigel (Sepic, Milan Italy) (2% w/w) and sodium valproate (3% w/w) in the opportune amount of distilled water with constant stirring at room temperature. The resulting gel was stored at room temperature for 24 h prior to use.

*In vitro skin permeation assay*: The analyses were carried out using a Franz type glass diffusion cell (LGA, Berkeley, CA, USA) assembled with membranes carrying stratum corneum and epidermis (SCE). Adult human skin samples (mean age 29±5 years) were obtained from abdominal reduction surgery. Subcutaneous fat was trimmed and the skin samples were immersed in distilled water at 60°C for 1 min; then stratum corneum and epidermis (SCE) were peeled off. The SCE samples were dried at room temperature in a desiccator maintained at approximately 25% relative humidity. The dried samples were wrapped in aluminium foil and stored at 4°C until use. Samples of dried SCE were rehydrated by immersion in distilled water at room temperature for 1 h before being mounted in Franz diffusion cells. The exposed skin surface area was 0.75 cm² and the receptor volume was 4.5 ml. The receiving solution, a solution of NaCl 0.9% (w/w), was stirred and thermo-stated at 35°C during the experiments. Skin barrier integrity of the SCE membranes used in this protocol was assessed by determining their tritiated water permeability coefficient (Kp). Kp values were found to be 1.58±0.3×10⁻³ cm h⁻¹ and were in accord with those previously reported in the corresponding literature (Bronaugh et al., 1986, J Invest Dermatol 87, 451-3). Sodium valproate (Valproate) was applied at 3% (w/w) topically to the skin surface. Each experiment was run in duplicate on three different skin donors. Samples of the receiving solution were withdrawn at different times during the experimental period (10 h); the sample volumes were replaced with the same amounts of fresh solution. All samples were analyzed for sodium valproate contents by means of an HPLC with spectrofluorimetric detection. The results were expressed as total amount permeated (mg).

*Quantitative determination of Sodium Valproate:* To determine sodium valproate, samples were derivatized with 4-bromomethyl-7-methoxycoumarin (BMMC). Cyclohexanecarboxylic acid was used as internal standard. The derivatization was carried out as previous reported (Bousquet et al., 1991, Pharmazie 46, 257-8). The analysis was performed with a chromatographic system consisting of a 1050 Hewlett Packard liquid chromatographer equipped with a 20 µl Rheodyne model 7125 injection valve (Rheodyne, Cotati, CA). The chromatographic analysis was carried out with a Hypersil ODS 5 µm column (Policonsult, Rome, Italy). The mobile phase was CH₃CN/H₂O (65/35 v/v) at a flow rate of 1 ml/min. Detection was performed at 310 nm.

### Results:

Figure 6 shows the results of an in vitro permeation assays. Adult human skin samples (skin surface area was 0.75 cm²) were pepared as described in the methods section. An appropriate amount of sodium valproate (Valproate) at 3% (w/w) was applied topically to the skin surface placed in the Franz type glass diffusion cells. Each experiment was run in duplicate on three different skin donors. Samples of the receiving solution were withdrawn at different times during the experimental period (10 h); the sample volumes were replaced with the same amounts of fresh solution. All samples were analyzed for sodium valproate contents by means of an HPLC with spectrofluorimetric detection. The results were expressed as total amount permeated (mg). VPA topically applied at 3% (w/w) permeates the skin in a time dependent manner. Thus, VPA is able to penetrate human skin effectively if applied topically.

### Example 6

VPA induces histone H4 acetylation in human immortalized keratinocytes (HaCat cells) in a concentration-dependent manner (Figure 7).

### Method:

*Westen blot:* HaCaT cells were seeded into 6-well culture dishes 24 hrs before treatment. Cultures were treated with the indicated concentrations of VPA and/or Tazarotene for 24 hours. Whole cell extracts were prepared by lysis of cells in RIPA buffer plus protease inhibitors for denaturing SDS gel electrophoresis on a 12% denaturing polyacrylamid gel. Acetylated histone H4 was detected by Western blot analysis using an anti-acetylated H4 antibody (clone T25; see patent application EP 02.021984.6) and as an equal loading control expression of β-actin was confirmed using an anti-actin antibody (Sigma, Cat.No. A5441 ).

### Results:

In Figure 7, the upper panel shows a representative anti-acetylated histone H4 immunoblotting of total cell lysate from HaCaT cells which were treated as indicated. The lower panel shows an anti-actin immunoblotting of a replica gel as loading control. Cells were plated in 6 well plates at day 0. Drugs were added on day 1, cells were lysed on day 2 with RIPA buffer plus protease inhibitors. 40 µg of total cell lysate were separated by 12.5% SDS PAGE and subjected to immunoblotting with the indicated antibodies. As can be seen in Figure 7, VPA induces histone H4 hyperacetylation in a dose dependent manner.

### Example 7

VPA topically applied at 3% (w/w) induces acetylation of histone H4 in nuclei of murine skin cells (Figures 8 and 9) and human Basal Cell Carcinoma (BCC) cells (Figure 10).

### Methods:

*Immunohistochemistry*: VPA (Valproate) topically applied at 3% (w/w) (see Example 5 for formulation details) or a placebo formulation were applied to the skin of athymic nude mice. Similarly, after giving informed consent, VPA applied at 3% (w/w), or a placebo formulation, were applied topically to BCC lesions of patients 16-20 hours before surgical excision. Cryostat skin sections of 3 µm thickness were fixed in acetone at room temperature for 10 min. Before staining with anti-acetylated H4 antibody (clone T25, see above) the tissue was blocked by preincubation with F(ab')2 fragments derived from goat anti-mouse immunoglobulin (Cappel) (1/10000 dilution in 50 mM Tris buffered saline, TBS) for 30 min. After rinsing with TBS for 10 min, the sections were incubated with anti-acetylated H4 antibody (clone T25) (1/4000 dilution in TBS) at room temperature for 1 hour. Then, the specimens were incubated with the rabbit anti-mouse serum and treated with APAAP (alkaline phosphatase anti-alkaline phosphatase) immunocomplex as described above. The positive staining was developed in red color with naphthol-AS-BI phosphate as a substrate, and New Fuchsin (Dako) as a chromogen. Pictures were taken with a digital camera connected to a light microscopy apparatus.

### Results:

Skin biopsies were frozen in liquid nitrogen immediately after excision. Immunohistochemistry was then performed on 3 µm sections as described in methods. Biopsies of skin topically treated with Placebo or VPA were processed in parallel, pictures were taken with a light microscopy apparatus connected to a digital camera under the same light intensity.
As can be clearly seen in Figures 8 and 9, topical VPA treatment induced a clear increase of histone H4 acetylation in skin cells of athymic nude mice and - most importantly - both in BCC cells and normal skin cells of patients (Figure 10). These data demonstrate that VPA topically applied to skin can effectively induce hyperacetylation of skin cells.

### Example 8

VPA potentiates the effects of Tazarotene in the treatment of superficial BCC's as can be seen in pictures of three representative patients before and after 6 weeks of topical treatment with Tazarotene applied at 0.1% (w/w) and VPA applied at 3 % (w/w) (Figure 11).

### Method:

After giving their informed consent, 10 patients (6 males, 4 females) mean age 68 years, were enrolled and treated topically with Tazarotene at 0.1% (w/w) and with VPA applied topically at 3% (w/w) once a day for 8 weeks. 10 lesions were treated: 8 Superficial BCCs and 2 pigmented BCC. 4 of these lesions had already been pre-treated with Tazarotene alone for at least 16-20 weeks, and appeared to be completely resistant to this treatment, i.e., no reduction of tumor diameter under Tazarotene treatment was observed. VPA was applied topically once a day 15-30 minutes before applying Tazarotene. Lesions were evaluated clinically and photographed every two weeks.

### Results:

The treatment of patients with BCC using topically applied VPA and Tazarotene led to a >50% regression (measured by diameter reduction) of all the lesions in 6 to 8 weeks of treatment. These results were observed both in the previously untreated patients, and in those previously exposed to Tazarotene alone. Interestingly, Tazarotene used alone has been found to induce regression in a number of BCC's (approximatively 50%), but regression is usually slow (12-24 weeks) and accompanied by several unwanted side effects (such as pain and itching). However, the topical treatment using VPA and Tazarotene in combination apparently accelerates the response to Tazarotene, reducing also the duration of the unwanted side effects caused by Tazarotene.

These results confirm that VPA can potentiate the effect of other compounds (such as Tazarotene) on tumor cells, particularly of tumors of the skin, and show that - in the case of Tazarotene-resistant BCC - VPA may convert a resistant form of a tumor into a sensitive one. Overall, these results confirm the efficacy of VPA in the topical treatment of skin hyperproliferative disorders.

### Example 9

| Formulation example (Ointment) | |
|---|---|
| White mineral oil | 20% |
| Cetyl Alcohol | 24% |
| Cetomacrogol 1000 | 6% |
| Valproic Acid | 3% |
| White vaseline | add to 100% |

### Example 10

| Formulation example (Paste) | |
|---|---|
| Zinc Oxide | 25% |
| Starch | 25% |
| Valproic Acid | 3% |
| White vaseline | add to 100% |

### Example 11

| Formulation example (Cream) | |
|---|---|
| White vaseline | 25% |
| Cetyl Alcohol | 10% |
| Tween 60 | 5% |
| Glycerin | 10% |
| EDTA | 0.2% |
| Sodium Valproate | 3% |
| Bidistilled Water | add to 100% |

## Claims

1. A topical pharmaceutical composition for the prevention or treatment of skin disorders, comprising:
(i) at least about 0.1 % of an active agent selected from the group consisting of VPA and pharmaceutically acceptable salts thereof, derivatives of VPA and pharmaceutically acceptable salts thereof; and
(ii) a dermatologically acceptable carrier.

2. A topical composition according to claim 1, wherein the concentration of the active agent is from about 0.1 % to about 6% of the composition.

3. A topical composition according to claim 1 or 2, further comprising retinoic acid or a derivative thereof.

4. A topical composition according to claim 3, wherein the concentration of retinoic acid or the derivative thereof is from about 0.01% to about 1% of the composition.

5. A topical composition according to claim 3 or 4, wherein the derivative of retinoic acid is selected from the group consisting of 9-cis retinoic acid, trans-retinoic acid, all-trans retinoic acid and Tazarotene.

6. A topical composition according to any one of the preceding claims, further comprising a chemotherapeutic drug.

7. A topical composition according to claim 6, wherein the chemotherapeutic drug is 5-Fluorouracil.

8. A topical composition according to claim 6 or 7, wherein the concentration of the chemotherapeutic drug is from about 0.1% to about 10% of the composition.

9. A topical composition according to any one of the preceding claims, wherein the dermatologically acceptable carrier is selected from the group consisting of creams, ointments, pastes and gels.

10. The use of VPA or a derivative thereof for the manufacture of a medicament for the prevention or treatment of a skin disorder, wherein a pharmaceutical composition comprising an active agent selected from the group consisting of VPA and pharmaceutically acceptable salts thereof, derivatives of VPA and pharmaceutically acceptable salts thereof is topically applied to the skin of an individual.

11. The use according to claim 10 wherein the treatment comprises administration of 0.5 to 10 mg/day per lesion of approximately 1 cm².

12. The use according to claim 10 or 11 wherein the skin disorder is a disease of the human skin in which induction of hyperacetylation of proteins has a beneficial therapeutic effect for patients.

13. The use according to any one of claims 10 to 12 wherein the skin disorder is selected from skin tumors, pre-neoplastic skin diseases, inflammations of the skin and/or mucosa and photoaging.

14. The use according to claim 13 wherein the skin disorder is a skin tumor selected from the group consisting of Basal Cell Carcinoma, Squamous cell carcinoma, Keratoakantoma, Bowen disease and cutaneous T-Cell Lymphoma.

15. The use according to claim 13 wherein the skin disorder is the pre-neoplastic skin disease Actinic keratosis.

16. The use according to claim 13 wherein the skin disorder is an inflammation of the skin and/or mucosa selected from the group consisting of Psoriasis, Ichtiosis and Acne.

17. The use according to any one of claims 10 to 16 wherein the active agent and a further active agent are topically applied in the same formulation or in separate formulations.

18. The use according to any one of claims 10 to 17 wherein a further inhibitor of histone deacetylases which is different to VPA is used.

19. The use according to claim 18 wherein the inhibitor of histone deacetylases which is different to VPA is selected from the group consisting of hydroxamic acid derivatives such as NVP-LAQ824, Trichostatin A (TSA), Suberoyl anilide hydroxamic acid, CBHA, Pyroxamide, Scriptaid, Cl-994, CG-1521, Chlamydocin, Biaryl hydroxamate, e.g., A-161906, Bicyclic aryl-N-hydroxycarboxamides, PXD-101, Sulfonamide hydroxamic acid, TPX-HA analogue (CHAP), Oxamflatin, Trapoxin, Depudecin, Apidicin, benzamides such as MS-27-27, pyroxamides and derivatives thereof, short chain fatty acids such as butyric acid, and derivatives thereof, e.g., Pivanex (Pivaloyloxymethyl butyrate), cyclic tetrapeptides such as trapoxin A, Depsipeptide (FK-228) and related peptidic compounds, Tacedinaline, MG2856, and HDAC class III inhibitors or SIRT inhibitors.

20. The use according to any one of claims 10 to 19 wherein VPA or the derivative thereof or the further inhibitor of histone deacetylases which is different to VPA as defined in claim 18 or 19, potentiates orally applied retinoid activity in neoplastic and non-neoplastic skin diseases including skin tumors; Basal Cell Carcinoma; Squamous cell carcinoma; Keratoakantoma; Bowen disease; cutaneous T-Cell Lymphoma; Actinic keratosis; Psoriasis; Ichtiosis; Acne; other inflammatory skin diseases, by topical application of VPA or the derivative thereof or the further inhibitor of histone deacetylases alone.

21. The use according to any one of claims 10 to 20 wherein VPA or the derivative thereof or the further inhibitor of histone deacetylases which is different to VPA as defined in claim 18 or 19, potentiates orally applied Vitamin D derivatives such as Tacalcitol and calcipotriol activity in neoplastic and non-neoplastic skin diseases including skin tumors; Basal Cell Carcinoma; Squamous cell carcinoma; Keratoakantoma; Bowen disease; cutaneous T-Cell Lymphoma; Actinic keratosis; Psoriasis; Ichtiosis; Acne; other inflammatory skin diseases by topical application of VPA or the derivative thereof or the further inhibitor of histone deacetylases alone.

22. The use according to any one of claims 10 to 21 wherein the treatment comprises administration of a topical pharmaceutical composition according to any one of claims 1 to 9.

23. The composition or use according to any one of the preceding claims wherein the derivative of VPA is a compound of formula I 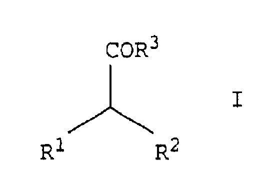 wherein R¹ and R² independently are a linear or branched, saturated or unsaturated, aliphatic C₃₋₂₅ hydrocarbon chain which optionally comprises one or several heteroatoms and which may be substituted, R³ is hydroxyl, halogen, alkoxy or an optionally alkylated amino group, or a pharmaceutically acceptable salt thereof.

24. The composition or use according to any one of the preceding claims, wherein R¹ and R² independently are a linear or branched C₃₋₂₅ hydrocarbon chain which optionally comprises one double or triple bond.

25. The composition or use according to any one of the preceding claims, wherein the VPA derivative is 4-yn-VPA or a pharmaceutically acceptable salt thereof.
